# EUROPEAN PATENT SPECIFICATION

(11) **EP 4 507 638 B1**
(45) Date of publication and mention of the grant of the patent: **23.09.2026**
(21) Application number: 23723293.9
(22) Date of filing: 13.04.2023
(51) Int. Cl.: A61F 7/00, A61H 9/00, A61H 23/04

(54) **WEARABLE THERAPY DEVICE FOR PROVIDING TEMPERATURE AND COMPRESSION THERAPY**
TRAGBARE THERAPIEVORRICHTUNG ZUR BEREITSTELLUNG EINER TEMPERATUR- UND KOMPRESSIONSTHERAPIE
DISPOSITIF DE THÉRAPIE TECHNOVESTIMENTAIRE SERVANT À FOURNIR UNE THÉRAPIE PAR TEMPÉRATURE ET COMPRESSION

(30) Priority: 13.04.2022 US 202263330429 P
(43) Date of publication of application: 19.02.2025
(73) Proprietor: Hyperice IP SubCo, LLC, Irvine, CA 92618 (US)
(72) Inventor: AGUIAR, Alexander Joseph, Irvine, California 92618 (US); KERTH, Trevor Austin, Irvine, California 92618 (US); KATZ, Anthony, Irvine, California 92618 (US); NORTHRUP, John Parker, Irvine, California 92618 (US)
(74) Representative: D Young & Co LLP
(86) International application number: PCT/US2023/018547
(87) International publication number: WO 2023/200987

(56) References cited:
- WO-A1-2014/001789
- WO-A1-2016/198904
- WO-A1-2023/004186
- US-A1- 2013 331 914
- US-A1- 2015 182 375
- US-A1- 2018 147 086
- US-B1- 10 470 922

## Description

### CROSS-REFERENCE TO RELATED APPLICATIONS

This application claims priority and benefit from U.S. Provisional Application No. 63/330,429, titled "Footwear for Providing Temperature and Compression Therapy" and filed on April 13, 2022.

### TECHNICAL FIELD

The present disclosure relates generally to the therapy field, and more specifically devices, systems, and methods for delivering compression therapy and/or temperature therapy.

### BACKGROUND

Conventional compression therapy devices are generally large devices that require a user to remain in a stationary position during use. Conventional temperature therapy devices such as electric heating pads, and ice packs have a limited duration of usefulness, e.g., re-usability, before they become ineffective and/or must be decommissioned. Such conventional temperature therapy devices can typically require pre-cooling or pre-heating, e.g., using a refrigerator, a microwave, among other external cooling/heating devices. Additionally, in using such conventional temperature therapy devices, an injured user can often be inconvenienced by having to be close to or make use of an external cooling/heating element to make effective use of the temperature therapy device. Therefore, typical temperature therapy devices may disrupt the required rest/recovery of a user, and can contribute to hindering of recovery times. Due to these and other the limitations of current compression or temperature therapy devices, it can further be difficult for these therapy devices to be made in smaller form factors and to be easily transported. Furthermore, conventional therapy devices often are limited to apply either only temperature therapy. Thus, an improved therapy device is needed. WO 2016/198904, US2013/331914, WO2014/001789, US2015/182375 WO2023/004186 and US2018/147086 are considered relevant prior art.

### SUMMARY

The invention is disclosed in independent claim 1. Preferred embodiments are disclosed in the dependent claims. Applicant has invented a technique for the improved, and in some embodiments combined, delivery of both temperature and compressive therapy. In some embodiments, compression can actually be used to facilitate a more therapeutically effective temperature therapy (e.g., by ensuring that the temperature delivery component maintains an appropriate amount of contact / force with the body of the user). Delivery of an appropriate amount of temperature therapy to achieve a desired outcome is a delicate balance. If not enough temperature therapy is delivered, then the desired physiological outcome may not occur. As one example, Applicant understood that for certain physical activities it can be desirable for the lubricating fluids between the skin surface and the muscle surface to be viscous. Such liquids tend to be thinner closer to the skin surface and more gel-like closer to the muscle surface. For certain physical activities, it can be desirable for the liquid proximate the muscle surface to be heated so as to convert from a gel-like substance to a more viscous substance. If the temperature therapy is delivered with too little heat or with too little force, then this result may not be achieved (or may take longer to achieve). On the other hand, if temperature therapy is too hot or delivered with too much pressure, it may be unbearable for the user to endure (and in some cases even result in burns, injury, etc.).

Applicant has discovered that strategic use of a compressive therapy force can enable improved delivery of temperature therapy and can be done in a manner so as to achieve the desirable results described in the foregoing paragraph. This differs from conventional devices that have both temperature and compressive aspects, because such devices focus on temperature and compressive therapy separately. Moreover, the delivery of temperature therapy in such devices in merely for user comfort or massage effect. That is starkly different from the inventive approach described herein, wherein compression is strategically applied at a pressure value determined to effective achieve therapeutic benefit (e.g., conversion of gel-like lubricating fluid to a more viscous state).

While this application may at times describe the concepts supported herein within the example embodiment of a footwear item, the skilled person will understand that invention can be implemented into any wearable garment for delivery of therapy to any body portion. As a few non-limiting examples: a vest for delivery of therapy to the chest / thoracic region, pants / shorts for delivery of therapy to the legs, waistband for delivery of therapy to the back, wraps for delivery of therapy to the arms or shoulders, among many other options).

Temperature therapy or "thermal therapy" (e.g., hot and cold therapy), as well as compression therapy (or compressive therapy) have been shown to be effective in injury recovery, helping to expedite the healing process while reducing pain, inflammation, and joint stiffness. Localized cooling can induce vasoconstriction with reflexive vasodilation and/or reduce bleeding, inflammation, metabolism, muscle spasm, pain, enzymatic activity, oxygen demand, and/or swelling in areas of the body affected by soft tissue trauma or injury. Localized heating can increase blood flow, decrease sensation of pain, increase local tissue metabolic rate, increase the rate of healing, and/or facilitate the stretching of tissue. Localized compression can increase blood flow through the veins of legs for preventing blood clots, help with treatment of lymphedema or chronic venous insufficiency, and reduce edema and aid return of venous blood to the heart.

The inventions disclosed herein include devices, for applying and delivering compressive therapy and temperature therapy.

In general, in one aspect, embodiments of the disclosure feature a wearable therapy delivery device. The device can include an inner layer configured to contact a body surface of a user; an outer layer; one or more temperature therapy modules enclosed between the inner layer and the outer layer proximate the inner layer; and one or more compressive therapy modules located proximate the one or more temperature therapy modules. In some cases, the one or more compressive therapy modules are configured to force the one or more temperature therapy modules toward the body surface of the user.

In some embodiments, the temperature therapy module includes at least one of a thermoelectric cooler (TEC) and a heating generation unit.

In some embodiments, the temperature therapy module is configured to operate in a range from 100°F to 140°F (37.8 °C to 60 °C).

In some embodiments, the compressive therapy module includes an inflatable bladder.

In some embodiments, the compressive therapy module is configured to operate in a range from 120 psi to 200 psi (827 kPa to 1378 kPa).

In the invention, the device further includes a spacer disposed between the compressive therapy module and the temperature therapy module. The spacer comprises at least one wing arranged about its perimeter. In some cases, the spacer includes 5 wings.

In some embodiments, the device further includes a control unit configured to control operation of the temperature therapy module and the compressive therapy module.

In some embodiments, the device includes a footwear item.

In general, in another aspect, embodiments of the disclosure feature a method for delivering a therapy. The method can include the steps of: providing a wearable therapy delivery device including an inner layer configured to contact a body surface of a user; an outer layer; one or more temperature therapy modules enclosed between the inner layer and the outer layer proximate the inner layer, and one or more compressive therapy modules located proximate the one or more temperature therapy modules, applying the inner layer of the device to the body surface; and activating the one or more temperature therapy modules and the one or more compressive therapy modules. In some cases, the one or more compressive therapy modules are configured to force the one or more temperature therapy modules towards the body surface of the user;

In some embodiments, the temperature therapy module includes at least one of a thermoelectric cooler (TEC) and a heating generation unit.

In some embodiments, the temperature therapy module is configured to operate in a range from 100°F to 140°F (37.8 °C to 60 °C).

In some embodiments, the compressive therapy module includes an inflatable bladder.

In some embodiments, the compressive therapy module is configured to operate in a range from 120 psi to 200 psi (827 kPa to 1378 kPa).

In some embodiments, the device further includes a spacer disposed between the compressive therapy module and the temperature therapy module. In some cases, the spacer comprises at least one wing arranged about its perimeter. In some cases, the spacer includes 5 wings.

In some embodiments, the device further includes a control unit configured to control operation of the temperature therapy module and the compressive therapy module.

In some embodiments, the device includes a footwear item.

The above and other preferred features, including various novel details of implementation and combination of events, will now be more particularly described with reference to the accompanying figures and pointed out in the claims. It will be understood that the particular systems and methods described herein are shown by way of illustration only and not as limitations. As will be understood by those skilled in the art, the principles and features described herein may be employed in various and numerous embodiments without departing from the scope of any of the present inventions. As can be appreciated from foregoing and following description, each and every feature described herein, and each and every combination of two or more such features, is included within the scope of the present disclosure provided that the features included in such a combination are not mutually inconsistent. In addition, any feature or combination of features may be specifically excluded from any embodiment of any of the present inventions.

### BRIEF DESCRIPTION OF THE DRAWINGS

The accompanying figures, which are included as part of the present specification, illustrate the presently preferred embodiments and together with the general description given above and the detailed description of the preferred embodiments given below serve to explain and teach the principles described herein.
FIG. 1A illustrates a block diagram of a temperature therapy device, according to some embodiments.
FIG. 1B illustrates a perspective view of an exemplary temperature modulation assembly, according to some embodiments.
FIG. 1C illustrates a view of an exemplary temperature modulation assembly, according to some embodiments.
FIG. 1D illustrates a temperature modulation assembly without its cover and cap, according to some embodiments.
FIG. 1E is an image of a temperature modulation assembly, according to some embodiments.
FIG. 2A illustrates a top view of a mounting plate of the temperature modulation assembly, according to some embodiments.
FIG. 2B illustrates a bottom view of the mounting plate, according to some embodiments.
FIG. 2C illustrates a cross-sectional view of the mounting plate, according to some embodiments.
FIG. 2D illustrates a zoom-in view of an opening of the mounting plate, according to some embodiments.
FIG. 3A illustrates a heat spreader of the temperature modulation assembly, according to some embodiments.
FIG. 3B illustrates a block diagram of a cross-section of the heat spreader, according to some embodiments.
FIG. 4 illustrates a thermoelectric cooler (TEC) of the temperature modulation assembly, according to some embodiments.
FIG. 5A illustrates a spacer of the temperature modulation assembly, according to some embodiments.
FIG. 5B illustrates a top view of the spacer as shown in FIG. 5A, according to some embodiments.
FIG. 5C illustrates a bottom view of the spacer as shown in FIG. 5A, according to some embodiments.
FIG. 5D illustrates a side view of the spacer as shown in FIG. 5A, according to some embodiments.
FIG. 5E illustrates a spacer of the temperature modulation assembly, according to some embodiments.
FIG. 5F illustrates a temperature modulation assembly including the spacer as shown in FIG. 5E, according to some embodiments.
FIG. 5G illustrates a temperature modulation assembly including the spacer as shown in FIG. 5E, according to some embodiments.
FIG. 6A illustrates a heatsink of the temperature modulation assembly, according to some embodiments.
FIG. 6B illustrates a cross-sectional view of the heatsink, according to some embodiments.
FIG. 6C illustrates a bottom view of the heatsink, according to some embodiments.
FIG. 7 illustrates a fan of the temperature modulation assembly, according to some embodiments.
FIG. 8A illustrates a cover of the temperature modulation assembly, according to some embodiments.
FIG. 8B illustrates a side view of the cover, according to some embodiments.
FIG. 8C illustrates a top view of the cover, according to some embodiments.
FIG. 8D illustrates another side view of the cover, according to some embodiments.
FIG. 8E illustrates a zoom-in view of a locking mechanism of the cover, according to some embodiments.
FIG. 9A illustrates a cap of the temperature modulation assembly, according to some embodiments.
FIG. 9B illustrates a top view of the cap, according to some embodiments.
FIG. 9C illustrates a bottom view of the cap, according to some embodiments.
FIG. 9D illustrates a cross-sectional view of the cap, according to some embodiments.
FIG. 10A illustrates an upper perspective view of a vibration pod, according to some embodiments.
FIG. 10B illustrates an exploded perspective view of the vibration pod showing the upper surfaces of the components, according to some embodiments.

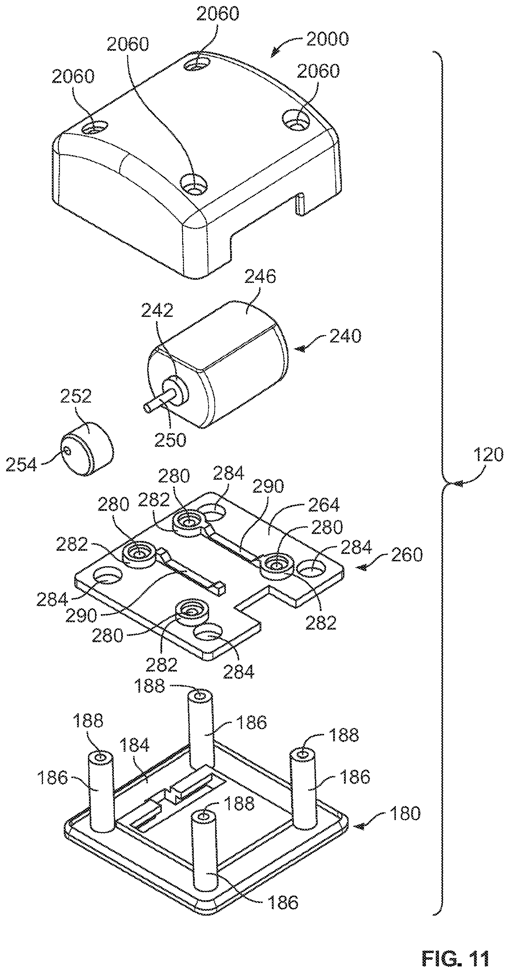

FIG. 11 illustrates an exploded perspective view of the vibration pod showing the lower surfaces of the components, according to some embodiments.

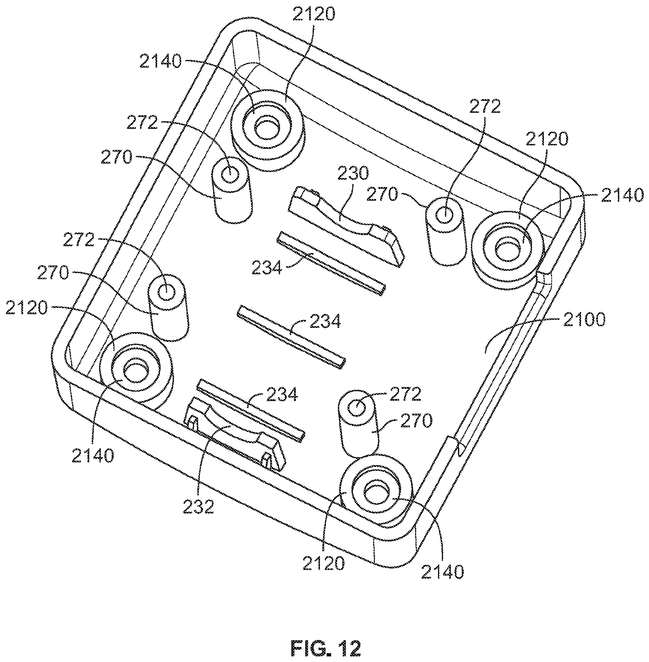

FIG. 12 illustrates an upper perspective view of the lower cover of the vibration pod rotated by a small angle to show addition features of the cavity of the lower cover, according to some embodiments;
FIG. 13 illustrates an exploded upper perspective view of a heating generation unit, according to some embodiments.
FIG. 14 illustrates a plan view of the electrical heating wire on the lower sheet of the heating generation unit, according to some embodiments.
FIG. 15A illustrates a footwear item, according to some embodiments.
FIG. 15B illustrates a footwear item, according to some embodiments.
FIG. 15C illustrates components of a footwear item, according to some embodiments.
FIG. 16 illustrates a view of a therapy device, according to some embodiments.
FIG. 17 illustrates a view of a footwear item, according to some embodiments.
FIG. 18 is a block diagram of an example computer system, according to some embodiments.

While the present disclosure is subject to various modifications and alternative forms, specific embodiments thereof have been shown by way of example in the drawings and will herein be described in detail. The present disclosure should be understood to not be limited to the particular forms disclosed, but on the contrary, the intention is to cover all modifications, equivalents, and alternatives falling within the scope of the present disclosure.

### DETAILED DESCRIPTION

Generally, the invention relates to devices, that include components or modules that can deliver temperature (e.g., hot, cold, and/or a desired temperature) therapy and compressive therapy to one or more portions of the body (e.g., foot, ankle, and/or lower extremity region) of a user.

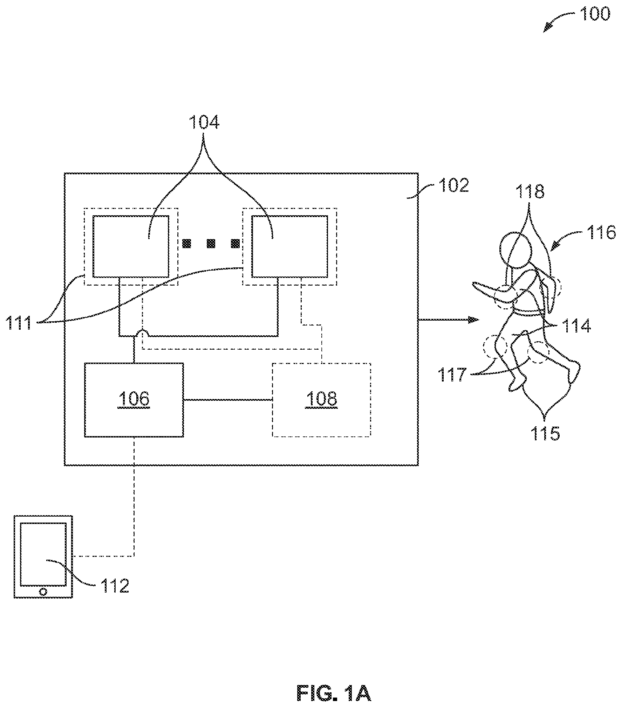

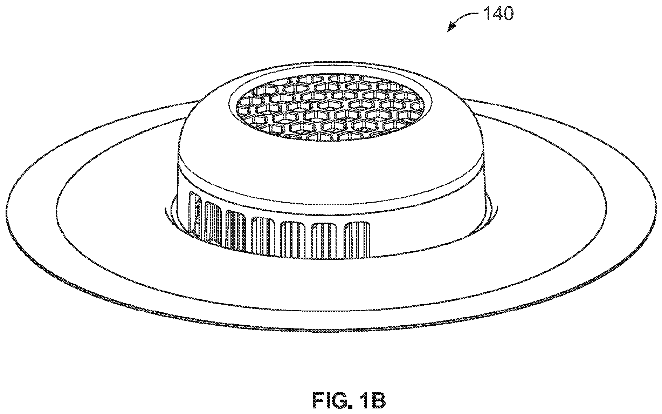

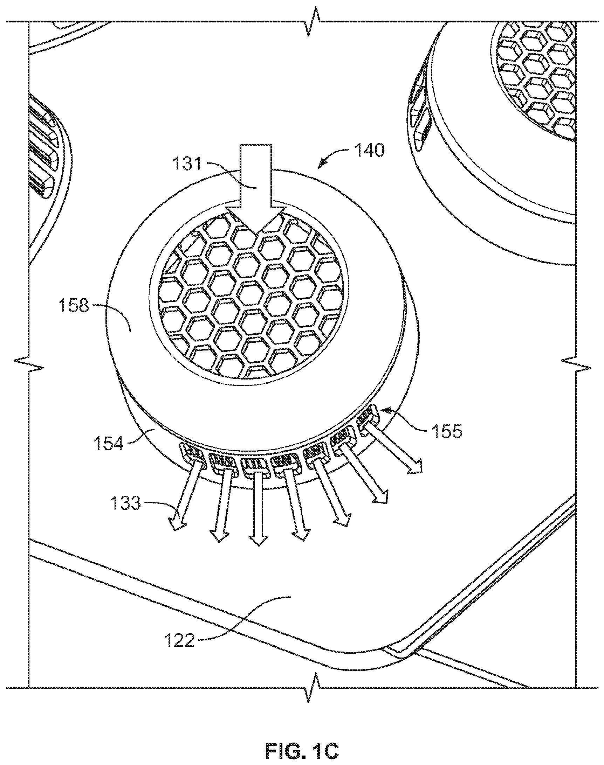

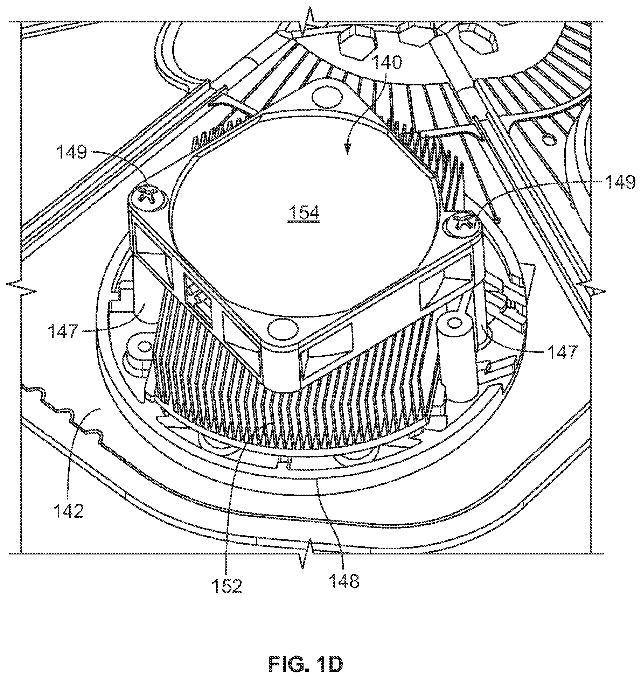

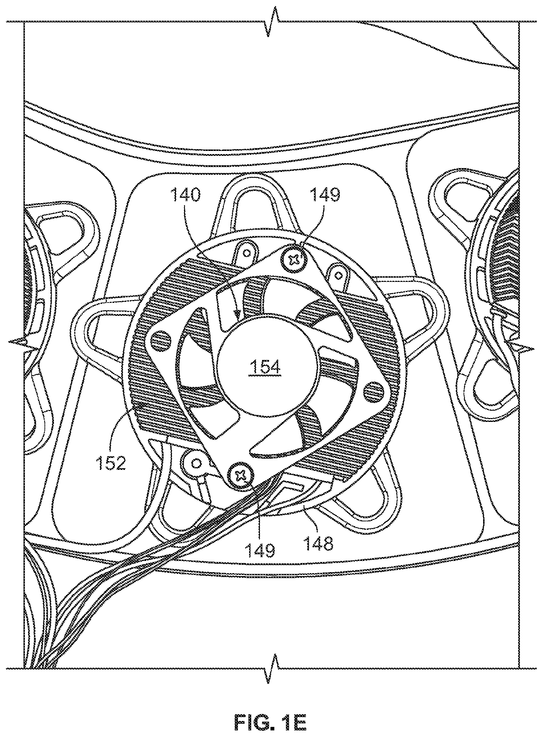

In some embodiments, temperature therapy as described herein is applied using a thermoelectric cooler (TEC) as described herein (e.g., in FIGS. 1-9), and for example, in U.S. Patent Publication No. 20190350752, U.S. Patent Application No. 17/308,012, and U.S. Patent Application No. 17/307,981.

In some embodiments, temperature therapy as described herein is applied using a polyimide or other type of heater as described, for example, in U.S. Patent Publication No. 20200170880, U.S. Provisional Patent Application No. 63/182,703, and U.S. Provisional Patent Application No. 63/243 546.

In some embodiments, compressive therapy as described herein is applied using a pneumatic system as described, for example, in U.S. Provisional Patent Application No. 63/248 077.

Advantageously, the therapy device can be worn for resting and recovering from a high ankle injury. Furthermore, the therapy device can deliver treatment that results in faster or improved recovery from an injury. For example, typical healing time for a broken ankle requiring wearing of a stabilizing boot can take 6 weeks or more. Use of the system and/or methods described herein can, in various embodiments, reduce healing time to a range from about 1 day to about 5 weeks (e.g., 2 weeks, 3 weeks, or 4 weeks). In addition, the therapy device as described herein can provide a level of mobility of the user's foot in use. Furthermore, the therapy device as described herein can provide a pre-exercise warm up device by delivering the heat and/or compression.

### Overview of Therapy Devices

In general, the therapy devices and systems as described herein include one or more modules that, upon activation, can delivery temperature (e.g., hot, cold, and/or a customized temperature) therapy and/or compressive therapy to a body region (e.g., a foot, ankle, and/or lower extremity region) of a user.

In some embodiments, each therapy module of the devices and systems as described herein can be selectively activated for delivering compressive therapy, thermal therapy, or combinations thereof.

In general, any number of modules for delivering thermal therapy can be used. In some embodiments, the therapy device as described herein includes 1, 2, 3, 4, 5, 6, or more modules for delivering thermal therapy.

In general, any number of modules for delivering compressive therapy can be used. In some embodiments, the therapy device as described herein includes 1, 2, 3, 4, 5, 6, or more modules for delivering compressive therapy.

In general, the therapy devices and systems can be configured to provide any suitable or desired level of therapy (e.g., temperature and/or compression) to a user. In some embodiments, the temperature therapy module is configured to operate in a range from about 50°F to about 150°F, (10 °C to 65.6 °C) from about 75°F to about 150°F, (23.9 °C to 65.6 °C) from about 100°F to about 140°F (37.8 °C - 60 °C). In some embodiments, the compressive therapy module is configured to operate in a range from about 100 psi to about 300 psi (689 kPa - 2068 kPa), about 110 psi to about 250 psi (758 kPa - 1723 kPa), or about 120 psi to about 200 psi (827 kPa - 1278 kPa).

In some embodiments, the therapy device as described herein further includes a battery pack. In some embodiments, the battery pack is enclosed in a box (e.g., a plastic box) mounted to the outside surface of the therapy device against the ankle that contacts the same area on the inside surface of the therapy device. In some embodiments, the box further includes an user interface to communicate with (e.g., displaying information to) the user. In some embodiments, the box encloses a control unit that is operatively connected to the battery pack and/or one or more therapy modules.

In use, a user can wear the therapy device by inserting a foot from an opening at the top portion of the therapy device down to the front portion of the therapy device. In some embodiments, the therapy device includes one or more zippers, laces, hook and loop (e.g., products of Velcro^{®}) and/or other suitable components to secure the therapy device to the foot area during the treatment.

In general, a method for delivering a thermal therapy or compressive therapy includes providing the therapy device to a user, applying the inner layer of the therapy device to the body surface of the user, and activating the one or more therapy modules to deliver thermal therapy or compressive therapy to the body surface. In some embodiments, the inner layer of the therapy device is applied to the body surface of the user using one or more zippers, laces, hook and loop (e.g., products of Velcro^{®}), and/or other suitable components.

Referring to FIG. 1A, a block diagram of a therapy device 100 is presented, according to some embodiments. In some embodiments, the therapy device 100 can include a multi-layer retention mechanism 102, a temperature modulation assembly 104 retained 111 by the multi-layer retention mechanism 102 (*e.g.,* one or more straps, buckles, fabric layers, etc.), and a control module 106 communicatively coupled to the temperature modulation assembly 104 and also retained by the multi-layer retention mechanism 102. In some embodiments, the multi-layer retention mechanism 102 can include one or more (e.g., all) flexible and/or elastic components of the therapy device 100. In an example, the multi-layer retention mechanism can include a flexible substrate. In some embodiments, the temperature modulation assembly 104 can include a fan, a heatsink and a thermoelectric cooler (TEC). In some embodiments, the temperature modulation assembly 104 can be configured to couple and secure a plurality of components of a therapy device 100 in a compact arrangement. In some embodiments, the temperature modulation assembly 104 can include, *e.g.,* be coupled to, a portion of the multi-layer retention mechanism 102. In some embodiments, the therapy device 100 can include one or more temperature modulation assemblies 104, as shown. In some embodiments, the therapy device 100 can also include a power supply module 108 retained by the multi-layer retention mechanism 102. The power supply module 108 can be electrically coupled to the temperature modulation assembly 104 and the control module 106. In some embodiments, the power supply module 108 may not be retained by the multi-layer retention mechanism 102. In an example, the power supply module 108 can instead be built into the control module 106. In one example, the power supply module 108 can be optional, where the control module 106 can include and/or perform all the functions of the power supply module 108. The therapy device 100 can also include a client application executing at a mobile device 112 in communication with the control module 106, and any other suitable components. In some embodiments, the therapy device 100 can include and/or can also be referred to as a wearable cooling and heating system.

Referring again to FIG. 1A, the therapy device 100 can deliver temperature regulated cold and/or hot therapy to a body region 114 of the user 116. In specific examples, the therapy device 100 can provide both cold and hot therapy the user 116, with rapid transitions between hot and cold therapy provision modes (*e.g.*, heating mode, cooling mode, etc.) of operation. In an example, the therapy device 100 can use the multi-layer retention mechanism 102 and the temperature modulation assembly 104 to provide the temperature therapy to a body region 114 of a user 116. The therapy device 100 can also function to regulate the temperature of the hot or cold therapy based on received control instructions (*e.g.*, from a mobile application-based controller, a computing device, a mobile computing platform 112, a client application execution thereon, etc.). The therapy device 100 can also function to monitor and/or track parameters of therapy provision, for example, the temperature of the hot or cold therapy being provided, the power and/or energy usage of the system during therapy provision, and/or any other suitable parameters. The therapy device 100 can also function to track user data such as frequency of use (*e.g.,* daily, hourly, monthly, etc.), duration of use (*e.g.,* total duration in minutes, duration on a per-operating-mode basis, duration on a per-contiguous-use basis, etc.) and therapy selection (*e.g.*, heat therapy, cold therapy), and provide tracked user data to an entity (*e.g.,* the user, a physical therapist associated with the user, etc.), in order to guide automated modes of therapy provision to the user.

The therapy device 100 can be worn by the user 116. In some embodiments, the therapy device 100 can be positioned at a region of a user (*e.g.,* a foot region 115, a knee region 117, a lower back region, an elbow region 118, etc.). In particular embodiments, the therapy device 100 is preferably a footwear that can be placed around a foot region of the user, arranging one or more temperature modulation subsystems proximal to a knee cap region of a user.

To effectively position and the temperature therapy components of a therapy device relative to a user, and provide temperature regulated therapy to a body region of a user, it can be beneficial to package together some inelastic and elastic components of the therapy device in a compact and/or portable arrangement.

### Modules for Delivering Thermal Therapy and/or Compression Therapy

In general, as discussed further below, the systems, methods, and devices as described herein can be or include: 1) a temperature therapy module (e.g., temperature modulation assembly such as a TEC and/or a heating generation unit) for applying or delivering thermal therapy, 2) a compressive therapy modules (e.g., a compression pad or inflatable bladder, and/or a vibration pod for applying or delivering compressive therapy, and/or 3) a control unit for operating a therapy device, and/or their combinations thereof.

In some embodiments, the devices, systems, and/or methods as described herein can be configured differently with additional or fewer modules or components. Or in some embodiments, the controller can be configured so as to only operate a certain subset of modules. For example, in some embodiments the therapy device may only apply temperature therapy or compressive therapy.

In some embodiments, the module delivers one or more levels of compressive therapy and/or temperature therapy. In some embodiments, a level of compressive therapy captures a part of the user's body area (e.g., the foot area, or ankle area, or other areas).

In some embodiments, the module as described herein can be disposed at any part of the therapy device. For example, a module can be disposed at inside surface the therapy device. In another example, a module can be disposed at outside surface the therapy device. In another example, a module can be disposed between the inside surface and the outside surface of the therapy device. In another example, a module is disposed at an area that is configured to contact the foot portion of the user. In some embodiments, the modules on the outside of the ankle portion of the user. In another example, a module is disposed at an area that is configured to contact the foot and ankle portion of the user.

### (i) Temperature Therapy Module

In general, the temperature therapy module (sometimes referred to herein as a temperature modulation assembly) can be configured to couple and secure a plurality of components for a temperature therapy device. The temperature modulation assembly can include one or more of a mounting plate, a heat spreader, a thermoelectric cooler (TEC), a spacer, a heatsink, a fan, a cover, and/or a cap which can be packaged together in a compact arrangement. In some embodiments, the mounting plate can be mounted to the spacer, and the heat spreader can be secured between the mounting plate and the spacer. In an example, the mounting plate can be an aluminum mounting plate. In some embodiments, the heat spreader can include 3 layers: a top layer including a first adhesive (, *e.g.,* a first silicone adhesive layer) layer, a middle layer including graphite/graphene and a bottom layer also including a second adhesive layer *(e.g.,* a second silicone adhesive layer). In some embodiments, a primer can be disposed on the bottom layer of the heat spreader, where the primer can be configured to bond a silicone adhesive on the mounting plate and/or on a silicone overmold insert to the bottom layer *(e.g.,* also a silicone adhesive layer) of the heat spreader. In some embodiments, the heatsink can be fitted and/or secured to the therapy device by mounting the fan to the spacer, where the heatsink can be secured between the fan and the spacer. In some embodiments, a cover can be placed over the spacer, heatsink and fan, where the cover can include vents (e.g., vents configured to allow airflow to and/or from the heatsink). In some embodiments, the cover can also secure a portion of a flexible fabric *(e.g.,* a top layer of a multi-layer retention mechanism) to the spacer. In some embodiments, a cap can be placed onto the cover, where the cap can include openings that are configured to allow for additional structural support and air intake into the temperature modulation assembly.

In some embodiments, a multi-layer retention mechanism is configured to retain components (e.g., flexible components) of the temperature therapy device. The multi-layer retention mechanism can include a top layer including control module, and a bottom layer including a silicone overmold insert. In some embodiments, the top layer can also include a flexible fabric and/or an elastic material. In an example, the top layer can include spandex. In some embodiments, the control module can include an electronics housing and electronic parts inside the electronics housing. In some embodiments, the bottom layer can include one or more bonding mechanisms, one or more structural support pieces, one or more straps and one or more locking mechanisms. In some embodiments, the bottom layer can include polyester and/or spandex. In some embodiments, the bottom layer can include polyester only. In some embodiments, the straps can be coupled indirectly to the bonding mechanism. In an example, the straps can be sewn into bottom layer adjacent the bonding mechanism to mechanically couple the straps, bottom layer and bonding mechanisms together. In some embodiments, the bonding mechanisms can include a flat spring that is flexible in one direction but inflexible in another, *e.g.*, perpendicular, direction. In some embodiments, the bonding mechanism can include metal and/or a metal spring. In some examples, the bonding mechanism can include a steel spring.

Referring to FIGS. 1B-1D, multiple views of a temperature modulation assembly 140 are presented, according to some embodiments. FIG. 1B shows a perspective view of the temperature modulation assembly. FIG. 1C shows a plan view of the temperature modulation assembly. FIG. 1D shows the temperature modulation assembly with the cap and cover removed. FIG. 1D depicts the configuration and coupling of the underlying components housed within the temperature modulation assembly 140.

As shown in FIGS. 1B and 1C, in some embodiments, the temperature modulation assembly 140 can include a cap 158 and cover 154. Also shown in FIG. 1C is the direction of the air flow used by the temperature modulation assembly 140 to regulate the temperature of the components housed within the temperature modulation assembly 140. In some embodiments, for air intake 131 into the temperature modulation assembly 140, air is pulled in though cap 158 by a fan into a heatsink, e.g., fan 154 and heatsink 152 shown in FIG. 1D. In some embodiments, for airflow outtake 133 of the temperature modulation assembly 140, the fan pushes air through the heatsink, and out of the temperature modulation assembly 140 through vents 155 of the cover 154. Furthermore, although the air flow is shown in one direction in the example of FIG. 1C, e.g., intake 131 through the cap 158 and exhaust through the vents 155, the air can flow in the opposite direction. For example, air can flow into the temperature modulation assembly through the vents 155 and exit the temperature modulation assembly through the cap 158.

FIG. 1D shows a plan view of the temperature modulation assembly without the cover and cap. FIG. 1E shows a perspective view of the temperature modulation assembly. As shown in FIGS. 1D and 1E, the temperature modulation assembly 140 can include a spacer 148, a heatsink 152, and a fan 154. The spacer 148 can also be referred to as a mounting component, among other terms. FIGS. 1D and 1E show an exemplary mounting configuration of the fan 154 to the spacer 148. In an example, the fan 154 can be secured by screws 149 inserted into columnal structures 147 of the spacer 148. Although not shown, the screws 149 can also be located through corresponding holes within the cover 158 of FIG. 1C. In some embodiments, as shown, the heatsink 152 can be secured between the fan 154 and the spacer 148. In an example, the heatsink 152 can be secured by a clamping force between the fan 154 and the spacer 148.

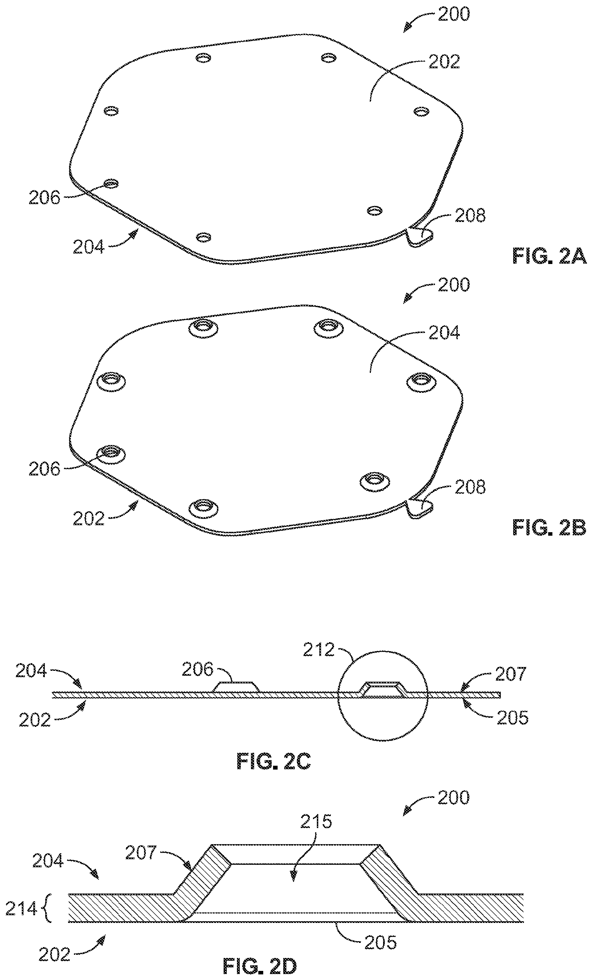

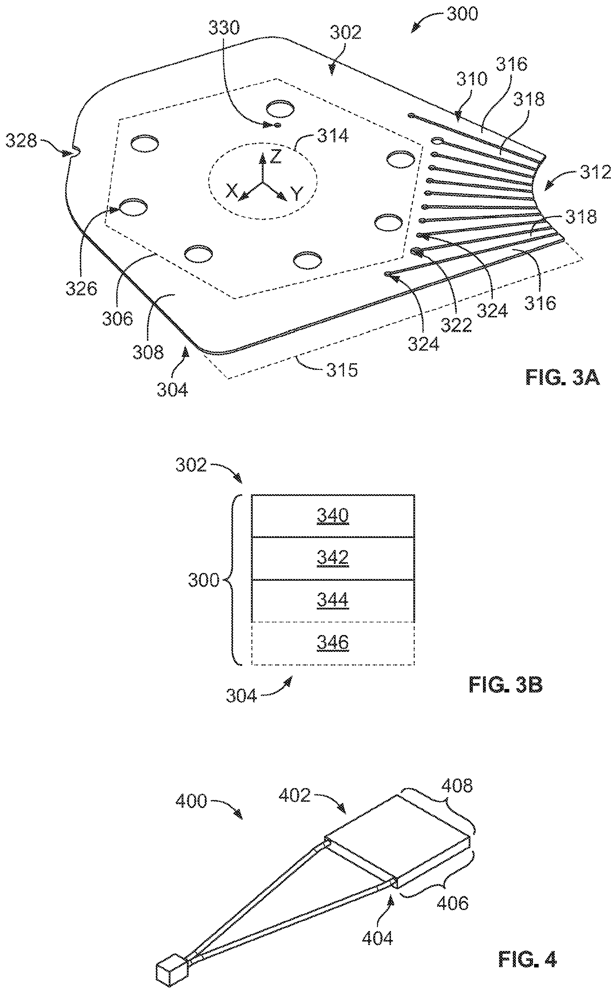

Each component of the therapy device in FIGS. 1 is described in detail below, according to some embodiments. For example, a mounting plate 124 is described in detail with reference to FIGS. 2A-2D. In another example, a heat spreader 126 is described in detail with reference to FIGS. 3A and 3B.

Referring to FIGS. 2A-2D, various views of a mounting plate of the temperature modulation assembly are shown, according to some embodiments. FIG. 2A shows a top view and 2B shows a bottom view of the mounting plate of the temperature modulation assembly. FIGS. 2C shows cross-sectional view of the mounting plate and FIG. 2D illustrates a zoom-in view of an opening of the mounting plate.

Referring to FIGS. 2A and 2B, a top view and a bottom view of a mounting plate of the temperature modulation assembly are presented respectively, according to some embodiments. As shown, the mounting plate 200 can have a top portion 202 and a bottom portion 204. FIG. 2A shows the mounting plate 200 from a top view, e.g., showing the top portion 202 of the mounting plate 200. FIG. 2B shows the mounting plate from a bottom view, e.g., showing the bottom portion 204 of the mounting plate 200. In some embodiments, the mounting plate 200 can include one or more openings 206. In some embodiments, the one or more openings 206 can be configured to receive one or more screws that can be used to mount the mounting plate 200 to a heat spreader and a spacer. In some embodiments, the mounting plate 200 can be cut into any suitable shape: circular, oval, polygonal, among other shapes. In an example, the mounting plate 200 can be in hexagonal shape as shown in FIGS. 2A and 2B. The mounting plate 200 can also include a tab feature 208. In an embodiment, the tab feature 208 can be in any shape: a triangular, circular, square, among other shapes. In an example, the tab feature 208 can be in a teardrop shape as shown. In some embodiments, the mounting plate 200 can include aluminum (e.g., anodized aluminum, 6061 aluminum, etc.). In some embodiments, the aluminum used in the mounting plate 200 is untreated, e.g., the aluminum is not anodized. In some embodiments, the mounting plate 200 can include any metal and/or alloy, for example, copper, steel, among other metals.

Referring to FIGS. 2C and 2D, respectively, a cross-sectional view of the mounting plate and a zoom-in view of an opening of the mounting plate are presented, according to some embodiments. In the views of FIGS. 2C and 2D, the top portion 202 of the mounting plate 200 is shown facing downward. In some embodiments, the mounting plate 200 can include a first adhesive 205 disposed over (e.g., on) a top portion 202 of the mounting plate 200. In some embodiments, the first adhesive 205 can include or be a double sided tape. In an example, the first adhesive 205 can include an acrylic on one side and a silicone adhesive on another side. In some embodiments, the acrylic side of the first adhesive 205 can be facing the top portion 202 of the mounting plate 200 and the silicon adhesive side can be facing a silicone overmold insert. In an example, the first adhesive 205 can include 3M^{™} tape 9731. In some embodiments, a peel off liner can be disposed over the first adhesive 205 to protect it during manufacturing. A second adhesive 207 can be disposed over (e.g., on) the bottom portion 204 of the mounting plate 200. In some embodiments, the bottom portion 204 of the mounting plate 200 can be coupled to the heat spreader 146 via the second adhesive 205. In an example, the second adhesive 205 can support adhering the mounting plate 200 to the heat spreader. A zoom-in view of an opening 212 from FIG. 2C is shown in FIG. 2D. Referring to FIG. 2D, in some embodiments, the mounting plate 200 can have a thickness 214 in a range of 0.2 mm to 1 mm. In an example, the mounting plate 200 can have a thickness 214 of approximately 0.40 mm. As shown in FIG. 2D, the first adhesive 205 can be disposed over the opening 212 (e.g., representative of the openings 206), where a gap 215 can be disposed between the opening 206 and the first adhesive 205. In some embodiments, the mounting plate 200 can be configured to anchor and/or stabilize the temperature modulation assembly 140. Additionally, when mounted together with the rest of the temperature modulation assembly components, the bottom portion 204 of the mounting plate 200 can be facing up toward a bottom portion of the heat spreader, as further described below.

Referring to FIGS. 3A and 3B, various views of a heat spreader of the temperature modulation assembly are shown, according to some embodiments. FIG. 3A show a heat spreader of the temperature modulation assembly. FIG. 3B shows a cross-sectional view of the heat spreader.

Referring to FIG. 3A, a heat spreader of the temperature modulation assembly is presented, according to some embodiments. The heat spreader 300 can have a top portion 302 and a bottom portion 304. An inner region 306 of the bottom portion 302 of the heat spreader 300 can be coupled to the bottom portion 204 of a mounting plate (e.g., the mounting plate 200 of FIGS. 2A-2D). When mounted together, at the inner region 306, the bottom portion 304 of the heat spreader 300 can be coupled to the bottom portion 204 of the mounting plate 200. In some embodiments, the heat spreader 300 can be configured to spread temperature in a horizontal and vertical direction, e.g., along x-, y- and z- directions. In an embodiment, the heat spreader 300 can be configured to provide for an efficient application of heat, which can enable the therapy device to deliver desired amounts and/or rates of hot or cold therapy using fewer TECs than would otherwise be required. In some embodiments, the heat spreader 300 can arrive as a roll at the beginning of a manufacturing process. In some embodiments, during manufacturing, the bottom portion 304 of the heat spreader 300 can include a liner which can later be removed to expose the adhesive disposed at the bottom portion 304 of the heat spreader 300.

Referring again to FIG. 3A, the heat spreader 300 can include one or more fingers 310 extending from one end 312 of the heat spreader 300. In one example, there can be 11 fingers 310 formed at one end 312 of the heat spreader 300. In some embodiments, at least one finger 310 can extend from one side 312, *e.g.,* a tapered side, of the heat spreader 300 toward a central portion 314 of the heat spreader. The fingers 310 can be cuts (e.g., narrow, elongated gaps) in the heat spreader material itself. In some embodiments, the fingers 310 can be configured to provide mechanical stress relief and/or strain relief when the therapy device is in use. In an embodiment, the average length of a finger 310 can be less than or equal to ½ and/or ¼ of the length of a side 315 of the heat spreader 300. In some embodiments, the lengths of the fingers 310 can be in a range of approximately 5-25 mm. In one example, the lengths of the fingers 310 can be in a range of approximately 10-20 mm (e.g., 10-15 mm). In some embodiments, the lengths of the fingers 310 are selected to provide as much surface area of the heat spreader as possible around a user's knee. In some embodiments, the one or more fingers 310 can include longer fingers 316 at an outer portion and shorter fingers 318 at an inner portion of the heat spreader 300. In some embodiments, the fingers 310 can include one or more openings 322, 324 at one end of each finger 310. In an embodiment, the openings 322, 324 can vary in size. In an example, one opening 324 at one end of one finger can have a diameter which is less than a diameter of another opening 322 at an end of another finger. In some embodiments, the openings can be configured to relieve mechanical stress from the fingers and/or at the one end 312 of the heat spreader 300.

Referring still to FIG. 3A, the heat spreader 300 can have one or more openings 326. The one or more openings 326 can be aligned with corresponding openings 206 of the mounting plate 200 from FIGS. 2A-2D. In some embodiments, the one or more openings 326 can be configured to receive one or more screws that can be used to mount the mounting plate 200 and heat spreader 300 to the spacer 148.

Referring yet again to FIG. 3A, the heat spreader 300 can include a notch 328. In an example, the notch 328 can be used to help an operator determine which temperature modulation assembly to attach to a corresponding heat spreader during manufacturing or fabrication.. In an example, although one heat spreader is shown, as described herein, multiple, *e.g.,* different/unique, heat spreaders and/or multiple, *e.g.,* different/unique, temperature modulation assemblies can be used. In some embodiments, a heat spreader 300 can have one or more notches. Each individual notch or group of notches can be different and/or unique from one another. In an example, as shown, the heat spreader 300 can include one notch 328. In another example, another heat spreader corresponding to another temperature modulation assembly can have two notches. In still another example, another heat spreader corresponding to another temperature modulation assembly can have three notches, and so on. Therefore, in some embodiments, the number of notches can determine which temperature modulation assembly is to be coupled to a corresponding heat spreader. For example, a first temperature modulation assembly can be mounted to the heat spreader 300 that includes the single notch 328, a second temperature modulation assembly can be mounted to another heat spreader that includes two notches, and so on. In some embodiments, there can be one to five notches, e.g., corresponding to up to five temperature modulation assemblies. As described above, in some embodiments, a therapy device can include one or more, *e.g.* greater than five, temperature modulation assemblies.

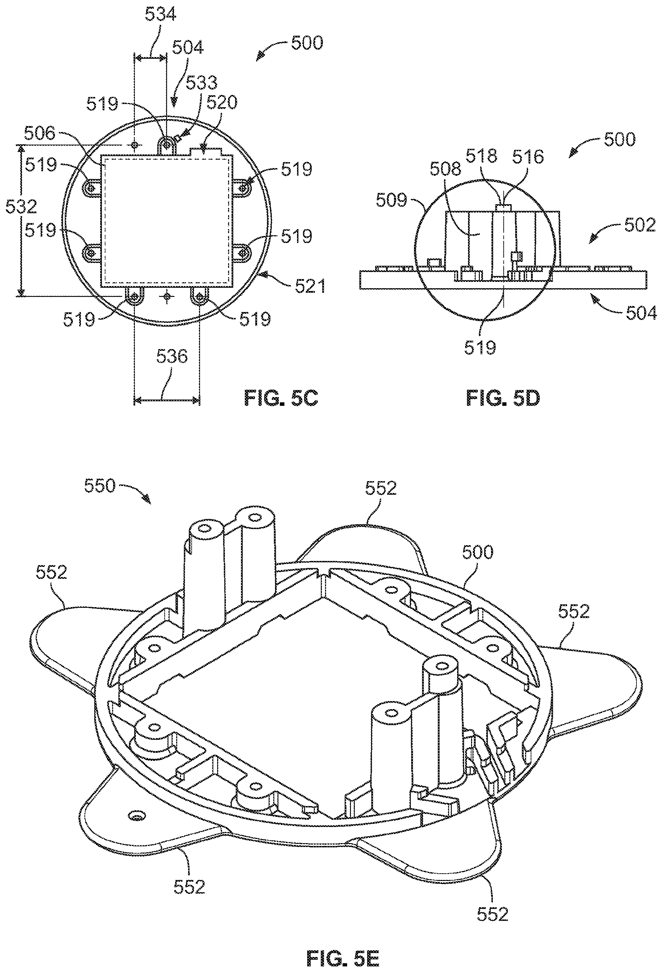

Referring still again to FIG. 3A, the heat spreader 300 can include an alignment opening 330. In an embodiment, the alignment opening 330 can be located adjacent to at least one mounting opening 326. In some embodiments, the alignment opening 330 can correspond to a bottom alignment feature 533 of the spacer 500 (*e.g.,* referring to FIG. 5C). In an example, the alignment opening 330 and bottom alignment feature 533 can be used to help an operator determine the proper alignment and/or placement between the spacer 500 and the heat spreader 300 during manufacturing and/or fabrication of a temperature modulation assembly. In an example, the bottom alignment feature 533 can fit into and/or align to the alignment opening 330.

Referring to FIG. 3B, a cross-section of the heat spreader is illustrated, according to some embodiments. In some embodiments, the heat spreader 300 can include 3 layers (340, 342, 344). In an example, the heat spreader can include a top layer 340, a middle layer 342 and a bottom layer 344. In some embodiments, the top layer 340 can be or include a first adhesive layer, the middle layer 342 can be or include a graphite/graphene layer and the bottom layer 346 can be or include a second adhesive layer. In some embodiments, the first adhesive layer and/or second adhesive layer can include a silicone adhesive. In some embodiments, the top layer 340 and/or bottom layer 344 can have a thickness in a range of approximately 8-12 micrometers. In some examples, the top layer and/or bottom layer can have thickness of approximately 10 micrometers. In some embodiments, a PET (polyethylene terephthalate) layer can be disposed over the top layer 340 and/or bottom layer 344. In an example, the middle layer 342 can include a graphene layer which includes a synthetic graphite sheet. In some examples, the middle layer 342 can include small particles *(e.g.,* of graphene). In some embodiments, the graphene layer can include a metal based powder for thermal energy transfer. In an example, the heat spreader 300 can include DSN5050-10DC10SB Synthetic Graphite Sheet from DASEN company.

Referring again to FIG. 3B, in some embodiments, a primer can be applied to the heat spreader. As shown, the primer 346 can be applied to the bottom layer 344 of the heat spreader 300. In some embodiments, applying the primer to the bottom layer 344 forms a primer layer 346 over the bottom layer 344. In some embodiments, the primer can be mixed with a catalyst, and subsequently applied (e.g., evenly spread) over the bottom layer 344 of the heat spreader 300. In an example, the primer can act as another layer disposed directly on the bottom layer 344. In some embodiments, in contrast to that shown in FIG. 3B, the primer can be applied to the top layer 340 of the heat spreader 300 rather than being applied to the bottom layer 344. In some embodiments, the primer can be applied to both the bottom layer 344 and the top layer 340 of the heat spreader. In some embodiments, the primer can be applied immediately after mixing with the catalyst. In an example, a ratio of 30:1 between the primer to the catalyst can be used. In some embodiments, the primer can be diluted using toluene. In some embodiments, a thin clothe and/or a latex glove can be used to apply the primer to the bottom layer 344 and/or top layer 340 of the heat spreader 300. In an example, the inventors found a cloth and/or a latex glove to be an effective application tool in comparison to a brush, where the brush can create markings, e.g., brush marks, on the primer after application. In some embodiments, the primer can be applied with a thickness of approximately 1 mm. In some embodiments, the entire bottom portion 304 and/or top portion 302 of the heat spreader can be covered by respective layers of the primer 346.

Referring still to FIG. 3B, subsequent to the mixing the primer with the catalyst and application of the primer to the bottom layer and/or top layer, the primer can be allowed to dry. In some embodiments, the primer can be allowed to dry for approximately 10-20 minutes. In some embodiments, once the primer is dry, the primer can be allowed to cure for any suitable amount of time before applying and/or adhering the mounting plate to the heat spreader 300. In some embodiments, the primer can be configured to allow the silicon adhesive on the mounting plate to uniformly adhere to the *e.g.,* the first or third layers 340, 344 of the heat spreader 300. In some embodiments, the primer can include a SilGrip PSA529 Silicone Pressure Sensitive Adhesive by Momentive.

Referring to FIGS. 3A, 3B and FIGS. 2A-2D, in some embodiments, the primer layer 346 can be used to couple the heat spreader 300 to a mounting plate (e.g., the mounting plate 200 of FIGS. 2A-2D) and to a silicone overmold insert. In an example, a silicone based adhesive disposed on the mounting plate 200 can be coupled directly to the primer layer 346. In some examples, the inner region 306 of the bottom portion 302 of the heat spreader 300 can be coupled to the bottom portion 204 of the mounting plate 200 via the primer layer 346 and via the silicone adhesive over the mounting plate 200. In a similar manner, in some embodiments, the heat spreader 300 can be coupled to the silicon overmold insert 142 at regions of the bottom portion 304 outside of the inner region 306 via a silicone adhesive on the silicone overmold insert 142 and via the primer layer 346. Therefore, in a same embodiment, the primer layer 346 can enable the coupling between the heat spreader 300, mounting plate 200 and silicon overmold insert 142 via the primer layer 346 and a silicone adhesive disposed over the mounting plate 200 and a silicone adhesive disposed over the silicone overmold insert 142. In specific example, the primer 346 can be configured to uniformly bond the heat spreader 300 to the mounting plate 200 and/or to the silicone overmold insert 142. In some embodiments, the configuration described above which is allows the heat spreader 300 and/or mounting plate 200 to bond to the silicone overmold insert 142 (e.g., silicone substrate) can be referred to as system for mounting rigid components to a silicone substrate for a temperature therapy device.

Referring to FIG. 4, a thermoelectric cooler (TEC) of the temperature modulation assembly is presented, according to some embodiments. In an embodiment, a TEC 400 can be selected based on its thermal conductivity rating. In an example, a TEC 400 having a high thermal conductivity rating, e.g., greater than or equal to the thermal conductivity of a ceramic material, can be used. The TEC 400 can have a top portion 402 and a bottom portion 404. In some embodiments, the length 406 of the TEC can be approximately equal to its width 408. In an example, the TEC 400 can be 40 mm in length 406 and 40 mm in width 408. A thermal grease can be disposed between a heat spreader and the TEC 400. In an example, a thermal grease with a high thermal conductivity, e.g., in the range of approximately 1-15 w/mk (for example, 1 w/mk), can be used. In an embodiment, a thermal grease from Halnziye company can be used.

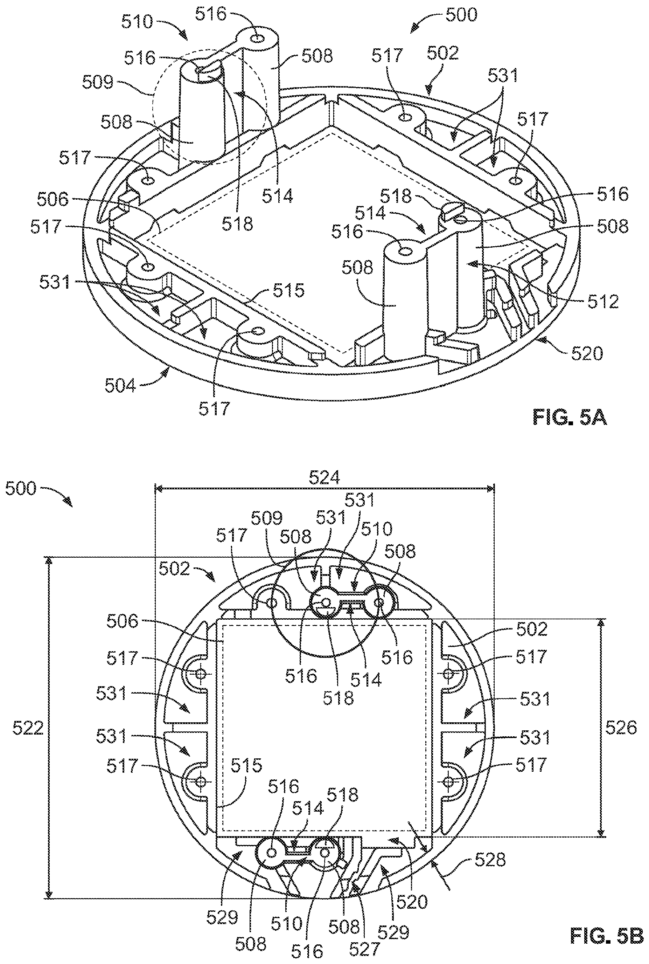

Referring to FIGS. 5A-5D, various views of a spacer are shown, according to some embodiments. In some embodiments, a spacer can include a mounting system for connecting elastic / flexible portions of the therapy device to inelastic, hard and/or solid elements. In an example, the spacer can include various mechanical features that are configured to mount/connect hard or solid elements to flexible objects. In one example, the spacer can also be called a mounting system. FIG. 5A illustrates a spacer of the temperature modulation assembly. FIG. 5B illustrates a top view of the spacer. FIG. 5C illustrates a bottom view of the spacer. FIG. 5D illustrates a side view of the spacer.

Referring to FIGS. 5A and 5B, multiple views of the spacer are presented, according to some embodiments. In some embodiments, the spacer 500 can include a top portion 502 and a bottom portion 504. In some embodiments, the spacer 500 can include a central opening 506 configured to receive a TEC (e.g., the TEC from FIG. 4). In some embodiments, the central opening 506 can include dimensions 524, 526 that allow the TEC to fit into the central opening 506. In an example, the central opening 506 can have a length 524 in a range of approximately 30-60 mm and width 526 in a range of approximately 30-60 mm. In some embodiments, the central opening 506 can include a shape a similar to and/or the same shape as the TEC, *e.g.,* a square opening. Although the central opening 506 can include a square opening, other shapes can be used such as a circular opening, polygonal opening, among others. As shown, the spacer 500 can have an outer diameter 522 in a range of approximately 50 -70 mm. In some embodiments, the spacer 500 can have an outer wall 528. In some embodiments, the outer wall 528 can have a thickness in the range of approximately 0.5 - 2.5 mm.

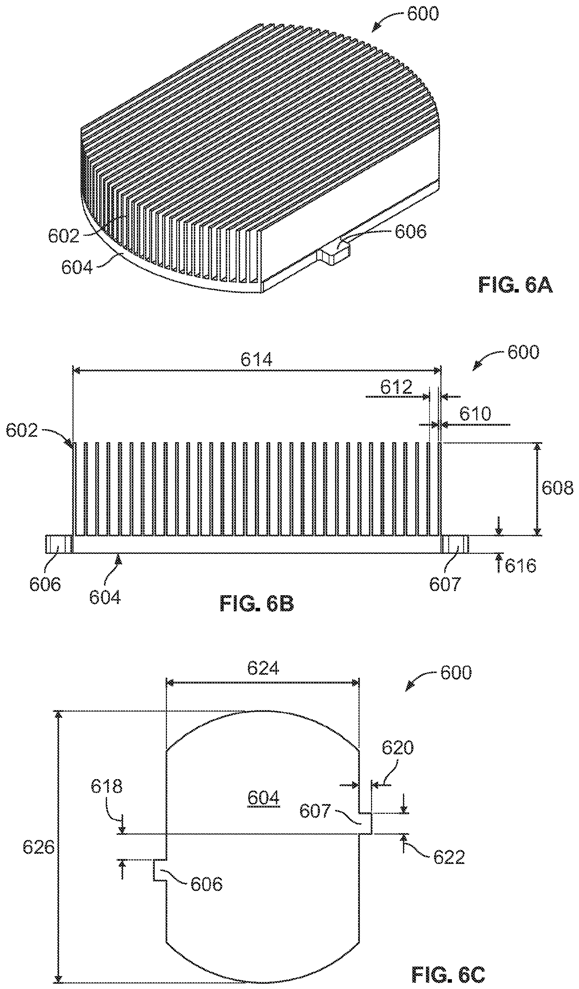

Referring again to FIGS. 5A and 5B, in some embodiments, the spacer 500 can include one or more columnal structures 508 extending from the top portion 502 of the spacer 500. As referred to herein, the one or more columnal structures 508 can be referred to as columnal structures or a plurality of columnal structures. In an example, the spacer 500 can include one or more columnal structures 508. An exemplary columnal structure is encircled in 509 of FIG. 5A and 5B. In some embodiments, a pair of columnal structures 510, 512 can be used, *e.g.,* one pair on either side of the spacer 500 as shown in FIGS. 5A and 5B. In an example, a first pair of columnal structures 510 and a second pair of columnal structures 512 are shown in FIGS. 5A and 5B. In some embodiments, the spacer 500 can include a notch 514. In an embodiment, the notch 514 can be configured to align the placement of a heatsink over the spacer 500 and secure the heatsink within the temperature modulation assembly *(e.g.,* the heatsink 152). In some embodiments, the notch 514 can be located between to columnal structures of a pair of columnal structures 510, 512, as shown in FIGS. 5A and 5B. In some embodiments, one or more notches 514 can be used. In an example, provided two pairs of columnal features 510, 512, there can be two corresponding notches, one for each per pair of columnal structures. In some embodiments, a heatsink can be placed over the spacer 500. In some embodiments, a tab of the heatsink (*e.g.*, as shown in FIGS. 6A-6C) can be aligned and/or placed into the notch 514 when positioning the heatsink over the spacer 500. In some embodiments, the notch 514 and tab of the heatsink can hold the heatsink in place over the spacer 500, resisting any movement of the heatsink. In an example, the notch 514 and tab combination can lock and/or hold the heatsink in place along a horizontal direction between the pairs of columnal structures 510, 512 of the spacer. In some embodiments, the heatsink can include one or more tabs. In an example, the heatsink can have two tabs and the two tabs can align with, and be placed into, two corresponding notches 514 of the spacer 500, *e.g.,* the two notches 514 shown in FIGS. 5A and 5B. In some embodiments, one or more notches 514 can be configured to keep heatsink from twisting in place and maintain a desired heatsink orientation, e.g., maintain direction or alignment of heatsink fins during and after assembly of the temperature therapy device.

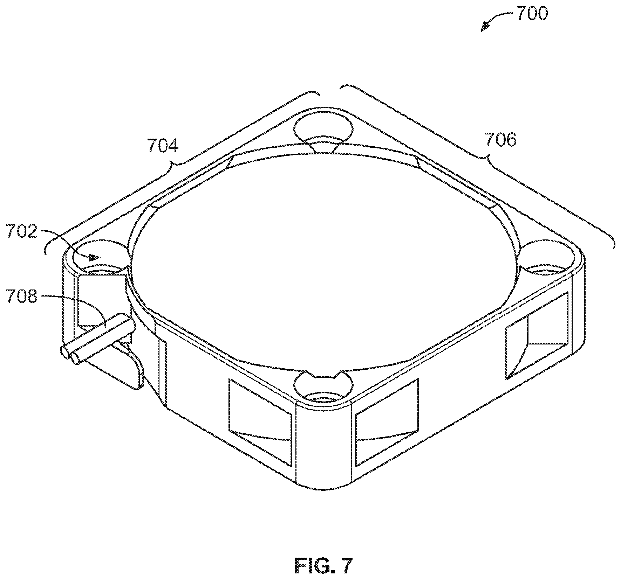

Referring still to FIGS. 5A and 5B, in some embodiments, each columnal structure 508 can include a top opening 516 configured to receive a screw to be inserted from the top portion 502 of the spacer 500. In some embodiments, the columnal structures 508 can be configured to receive a screw for mounting a fan. In an example, the columnal structures 508 can be configured to receive a screw for mounting the fan from FIGS. 1 and 7. In an example, a screw can he placed through a corresponding opening in the fan and into a top opening 516. In some embodiments, one or more top openings 516 can be used. In an example, there can be a top opening 516 corresponding to each columnal structure 508. In some embodiments, a top alignment feature 518 can be located at a top end of one or more columnal structures 508 next to the top opening. In some embodiments, the top alignment feature 518 can be configured to ensure that an operator inserts a screw in a top opening 516 in the correct direction and/or configuration. In example, an operator can refer to the top alignment feature 518 when placing a screw in a top opening 516 for mounting the fan. In some embodiments, not all columnal structures 508 can include a top alignment feature 518. In an example, only columnal structures 508 which are used for mounting a fan can include a top alignment feature 518. In an embodiment, an operator can be instructed to locate the top alignment feature 518 and only place a screw (*e.g.,* for mounting a fan) where the top alignment feature 518 is located. In some embodiments, the alignment features 518 located on a first pair of columnal structures 510 may not be aligned with another alignment feature 518 located on a second pair of columnal featured 512. In an example, an alignment feature 518 can be located on a first columnal structure of the first pair of columnal structures 510 and another alignment feature 518 can be located on a second, different, columnal structure of the second pair of columnal structures 512 as shown in FIGS. 5A and 5B. In an embodiment, the top alignment feature 518 can include various shapes. In some embodiments, the top alignment feature 518 can have various shapes such as circular, square, rectangular, oblong, polygonal, among others. In an example, as shown in FIGS. 5A and 5B, the top alignment feature 518 can include a half circle and/or half-moon shape. Thus, in some embodiments, the top alignment feature 518 can be configured to assist an operator in the location and/or placement of a fan. In some embodiments, the fan can be mounted over the heatsink and spacer using one or more screws to keep the heatsink in place, *e.g.,* as described above. For example, during assembly of the mounting system, the heatsink can move and/or twist. Thus, in an example, the fan can be mounted over the heatsink and the spacer such that the heatsink is held in place by the fan. In the same example, the fan can keep the heatsink in place and prevent the heatsink from moving during and after assembly of temperature modulation assembly. In some embodiments, not all top openings 516 may correspond or be located at a columnal structure 508. In an example, some top openings 517 can be located on lower sides of the top portion 502.

Referring yet again to FIGS. 5A and 5B, in an embodiment, the spacer 500 can include one or more wire management features. In an example, the spacer 500 can include wire management features configured to receive at least one wire from a temperature sensor, TEC and/or fan. In some embodiments, the wire management features can include ravines, wells, burrs and/or cut-outs built into the spacer 500 that include the dimensions of the wires for the electronics, e.g., from the temperature sensor, TEC and/or fan. In an example, the wire management features can be configured to receive one or more wires and can secure the wires in place during and after assembly of the temperature therapy device. In an embodiment, the spacer 500 can include a first wire management feature 527 that is configured to receive and secure one or more wires from a TEC. In an embodiment, the spacer 500 can include a second wire management feature 529 that is configured to receive and secure one or more wires from a fan. In an example, the wire management feature 529 for the fan can act like a hook and hold the wires from the fan in place.

Referring again to FIGS. 5A and 5B, in an embodiment, the spacer 500 can include one or more receiving features for various elements of the temperature modulation assembly. In some embodiments, the spacer 500 can include a receiving portion in a form of a cut-out 520 for a temperature sensor. In an example, the temperature sensor can be coupled to the TEC and the temperature sensor can fit into the cut-out 520 of the spacer 500. The spacer 500 can include a plurality of receiving portions 531, e.g., which also can be referred to as channels, to receive corresponding alignment features of the top layer. Also, the spacer 500 can include an edge 515 adjacent to the alignment features 531. In some embodiments, the edge 515 of the spacer 500 can be aligned to a flat edge of the alignment feature of the top layer.

Referring to FIG. 5C, a bottom view of the spacer is presented, according to some embodiments. In some embodiments, the spacer 500 can include one or more bottom openings 519 located at the bottom portion 504 of the spacer 500, the bottom openings 519 can be configured to receive screws from the bottom portion 504 of the spacer 500. In some embodiments, the mounting plate from FIGS. 2A-2D can be mounted to the bottom portion 504 of the spacer 500 by inserting one or more screws through the bottom openings 519 in the spacer 500 and corresponding openings in the mounting plate. In some embodiments, the spacer 500 can include 7 bottom openings 519. In an example, the sum of the bottom openings 519 can add up to an odd number, *e.g.*, the number of bottom openings 519 are not designed to be symmetric or even. In some embodiments, the bottom openings 519 can have an asymmetrical configuration. In an embodiment, the asymmetrical configuration of the bottom openings 519 can ensure that the mounting plate is mounted in a particular order, *e.g.,* to prevent an operator from mounting the mounting plate incorrectly or opposite to the intended configuration. In some embodiments, the bottom portion 504 of the spacer 500 can have embossed and/or chamfered edges 521. In some embodiments, the edges 521 of the bottom portion 504 can be configured to receive and/or align to the edges of the mounting plate. In some embodiments, the distance between opposite bottom openings 532 can be in a range of approximately 30-60 mm. In some embodiments, the distance between adjacent bottom openings 534, 536 can be in a range of approximately 5 - 30 mm.

Referring again to FIG. 5C, in some embodiments, the spacer 500 can include a bottom alignment feature 533. In some embodiments, the bottom alignment feature 533 can be located adjacent to at least one bottom opening 519. In some embodiments, the bottom alignment feature 533 can correspond to an alignment opening 330 of the heat spreader 300 (*e.g.,* of FIG. 3A and 3B). In an example, the bottom alignment feature 533 and alignment opening 330 can be used to help an operator determine the proper alignment and/or placement between the spacer 500 and the heat spreader 300 during manufacturing and/or fabrication of a temperature modulation assembly. In an example, the bottom alignment feature 533 can fit into and/or align to the alignment opening 330.

Referring to FIG. 5D, a side view of the spacer is presented, according to some embodiments. A cross-sectional view of the columnal structures 508 from FIGS. 5A and 5B is shown in FIG. 5D. As shown, an exemplary columnal structure is encircled in 509 of FIG. 5D. Also, a side view of the top alignment feature 518 is shown in FIG. 5D. Additionally, FIG. 5D shows that the top opening can 516 extend through the spacer and meet a corresponding bottom opening 519. In some embodiments, each of the top openings 516 can have a corresponding bottom opening 519 *e.g.,* from FIGS. 5A-5D. In some embodiments each of the top and bottom openings can 516, 519 be a through-hole, e.g., the openings can extend from the top opening 516, through the spacer 500 and out through a corresponding bottom opening 519.

Referring still again to FIGS. 5A-5D, in some embodiments, the spacer can include a material such as a plastic, resin and/or fireproof plastic/resin, among other materials. In an example, the spacer can include a material selected from the group consisting of Nylon 66, Dupont 801, and Dupont 2801.

FIG. 5E illustrates a spacer 550 of the temperature modulation assembly, according to some embodiments. In some embodiments, as shown in FIG. 5E, the spacer 550 includes a similar structure as the spacer 500, and further includes one or more wings 552 disposed at the outer perimeter of the spacer 500.

Applicant identified that in some instances, especially when a compressive therapy module was activated that was not in direct contact with the temperature therapy module (e.g., when a bladder as described in U.S. Patent Application No. 17/384,501, is inflated), it can create a reduction in contact force (or in some cases a gap) between the temperature therapy module and the body surface. This is because the compressive force applied directly to the body surface is not also applied correspondingly to the temperature therapy module. To address this situation, Applicant incorporated a feature / protrusion into the temperature therapy module that makes direct contact with the compressive therapy module, such that the temperature therapy module and the body surface are compressed at the same time. In general, the feature / protrusion can take any suitable form, one example of which are the wings 552 shown in FIG. 5E. In use, the wings 552 can enable and/or stabilizing the temperature modulation assembly towards the user's body to deliver temperature therapy.

The wings can be in any number and be arranged in any suitable orientation (e.g., symmetric or non-symmetric, equidistant or non-equidistant). In some embodiments, the distance between any two adjacent wings 552 are the same. Accordingly, the spacer 550 can have a radial symmetry.

In some embodiments, the spacer 550 includes one or more (e.g., 1, 2, 3, 4, 5, 6, 7, 8, 9, 10, or more) wings. In some embodiments, 5 or more wings are preferable.

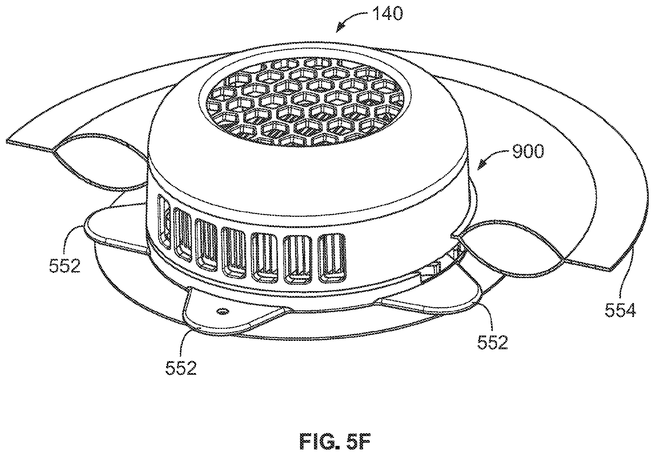

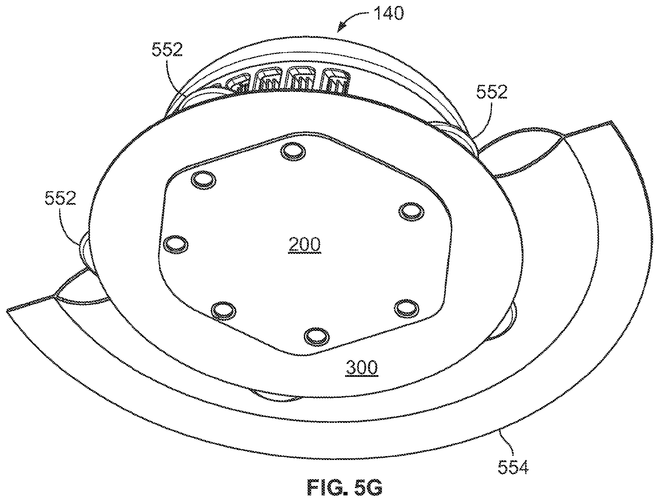

FIGS. 5F and 5G are various views of a temperature modulation assembly including the spacer 550, according to some embodiments. FIG. 5G also shows the additional components such as the mounting plate (e.g., mounting plate 200), and/or the heat spreader 300, which are assembled with the spacer 550. In some embodiments, the mounting plate includes any suitable shape: circular, oval, polygonal, among other shapes.In some embodiments, the heat spreader includes any suitable shape: circular, oval, polygonal, among other shapes. In some embodiments, the temperature modulation assembly 140 further includes a ring-shape component 554 that is operatively connected to the outer perimeter of the cap (e.g., cap 900) of the temperature modulation assembly.

Referring to FIGS. 6A-6C, various views of a heatsink are shown, according to some embodiments. In some embodiments, the heatsink can include a component and/or material configured to draw heat away the TEC and/or other elements of the temperature modulation assembly. FIG. 6A illustrates a heatsink of the temperature modulation assembly. FIG. 6B illustrates a cross-sectional view of the heatsink. FIG. 6C illustrates a bottom view of the heatsink.

Referring to FIG. 6A, a heatsink is presented, according to some embodiments. In some embodiments, the heatsink 600 includes a plurality of fins 602 extending from a base portion 604 of the heatsink 600. In some embodiments, the plurality of fins 602 can be formed through a skiving technique. In some embodiments, the plurality of fins 602 can be referred to as skived fins. In some embodiments, in contrast to using extrusion which is one way conventional heatsinks are formed, the entire heatsink 600 can be formed using a skiving technique. In some embodiments, the heatsink 600 can be referred to as a skived heatsink. In an example, a metal work skiving process can be used to form heatsink 600 and/or the plurality of fins 602. As referred to herein the plurality of fins 602 can also be referred to individually, e.g., each fin 602 or as one or more fins 602. In some embodiments, the heatsink 600 can include a first tab 606. In some embodiments, one or more tabs can be used as shown in FIGS. 6B and 6C.

Referring to FIG. 6B, a cross-sectional view of the heatsink is presented, according to some embodiments. To maximize the heat dissipation for a temperature therapy device, it can be useful to form the fins 602 as thin as possible. In some embodiments, each fin 608 can include a thickness 610 in a range of approximately 0.2 - 0.4 mm. In an example, each fin 608 can have a thickness 610 of approximately 0.3 mm. In some embodiments, each fin 602 can have a height 608 in a range of approximately 9 - 11 mm. In an example, each fin 602 can have a height 608 of approximately 10 mm. In some embodiments, the distance 612 between each fin 608 can be in a range of approximately 0.80 mm - 1.0 mm. In an example, the distance 612 between each fin 608 can be approximately 0.95 mm. In some embodiments, the distance 614 between a fin on one side of the heatsink to another fin on another opposite side of the heatsink can be in a range of 39 - 41 mm. In some embodiments, the distance 614 between the fin on one end to the last fin on the corresponding opposite end of the heatsink can be approximately 40.3 mm. In some embodiments, the heatsink 600 can include approximately 10 - 50 fins. In an example, the heatsink 600 can include 33 fins. In some embodiments, the heatsink can have 27 fins. In some embodiments, the base 604 of the heatsink 600 can have a thickness 616 in a range of approximately 1.5 - 2.5 mm. In an example, the base 604 of the heatsink 600 can have a thickness 616 of approximately 2.0 mm. In some embodiments, the base 604 can have a substantially smooth bottom surface. The heatsink 600 can also have tabs 606, 607, which are discussed in FIG. 6C below.

Referring to FIG. 6C, a bottom view of the heatsink is presented, according to some embodiments. The heatsink 600 can include a first tab 606 and a second tab 607, as shown. In embodiment, as discussed above, the tabs 606, 607 of the heatsink 600 can be configured to align and/or fit into one or more notches 514 of the spacer 500 of FIGS. 5A - 5D. As shown, the tabs 606, 607 may not be aligned along a horizontal direction of the figure. In some embodiments, the first and second tabs 606, 607 can be offset from one another. In some embodiments, the first tab 606 can be offset from the second tab 607 by a distance 618 in a range of approximately 4.5 - 6.5 mm. In an example, the first tab 606 can be offset from the second tab 607 by a distance 618 of approximately 5.6 mm. In some embodiments, the tabs 606, 607 can extend 620 from the heatsink 600. In some embodiments, the extension 620 of the tabs 606, 607 from the heatsink 600 can be in a range of approximately 1.5 - 3.5 mm. In an example, the extension 620 of the tabs 606, 607 from the heatsink 600 can be approximately 2.70 mm. In some embodiments, the first and second tab 606, 607 can have a width 622 in a range of approximately 3.5 - 5.5 mm. In an example, the first and the second tab 606, 607 can have a width 622 of approximately 4.4 mm. In some embodiments, the heatsink 600 can have a width 624 in a range of approximately 35 - 45 mm. In an example, the heatsink 600 can have a width 624 of approximately 40.70 mm. In some embodiments, the heatsink 600 can have a length 626 in a range of approximately 50 - 60 mm. In an example, the heatsink 600 can have a length 626 of approximately 57.50 mm.

Referring to FIGS. 6A-6C, in some embodiments, the heatsink can include aluminum. In an example, the heatsink can include anodized aluminum. In some embodiments, the heatsink can include aluminum 6063.

Referring to FIG. 7, a fan of the temperature modulation assembly is presented, according to some embodiments. In some embodiments, the fan 700 includes a plurality of openings 702. In some embodiments, the openings 702 can be configured to receive a screw for mounting the fan to the spacer described in FIGS. 5A-5D. In some embodiments, the width 704 of the fan 700 can be in a range of approximately 35 - 45 mm. In an example, the width 704 of the fan 700 can be approximately 40 mm. In some embodiments, the length 706 of the fan 700 can be in a range of approximately 35 - 45 mm. In an example, length 706 of the fan 700 can be approximately 40 mm. The fan 700 can include wires 708 for electrical power.

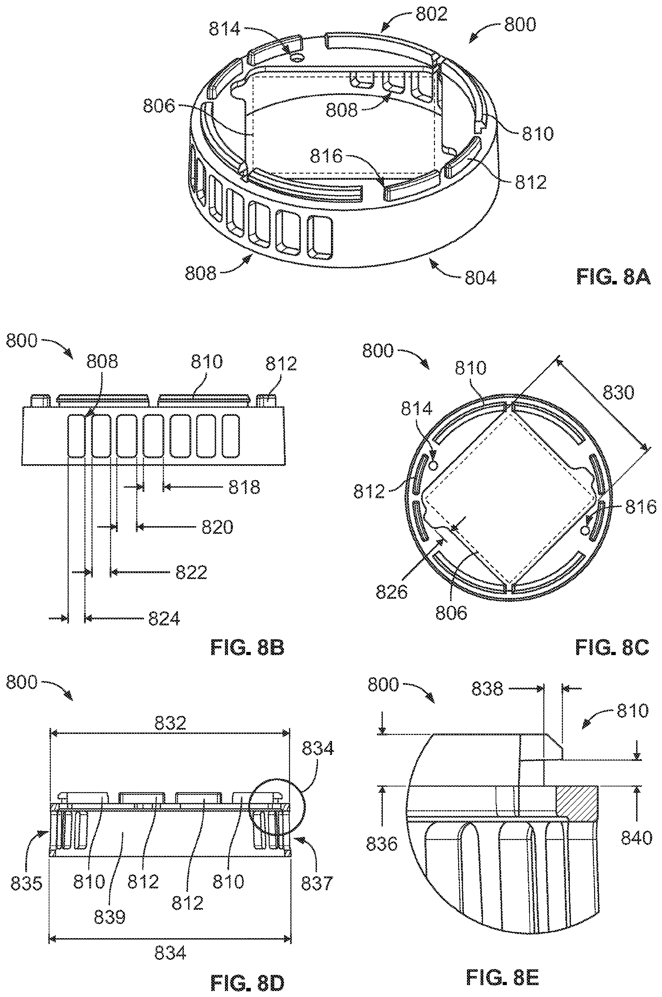

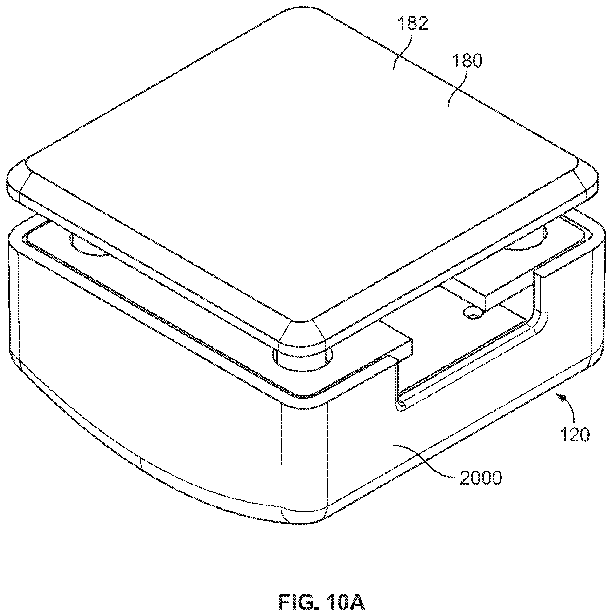

Referring to FIGS. 8A-8E, various views of a cover are presented, according to some embodiments. In some embodiments, the cover can be an intermediate structure configured to enclose and/or house the spacer from FIGS. 5A-5D, heatsink from FIGS. 6A-6C and the fan from FIG. 7. The cover can also be configured to secure the top layer of FIGS. 10A-10D to the spacer of FIGS. 5A-5D. FIG. 8A illustrates a cover of the temperature modulation assembly. FIG. 8B illustrates a side view of the cover. FIG. 8C illustrates a top view of the cover. FIG. 8D illustrates another side view of the cover. FIG. 8E illustrates a zoom-in view of a locking mechanism of the cover.

Referring to FIG. 8A, a cover of the temperature modulation assembly is presented, according to some embodiments. In some embodiments, the cover 800 can include a top portion 802 and a bottom portion 804. In some embodiments, the cover 800 can include a central opening 806 (e.g., similar to the central opening of the spacer in FIGS 5A-5D). In some embodiments, the central opening 806 can include a shape a similar to and/or the same shape as the fan of FIG. 7, *e.g.,* a square opening. Although the central opening 806 can include a square opening, other shapes can be used such as a circular opening 806, polygonal opening, among other shapes. In some embodiments, the cover 800 can be positioned over a spacer, heatsink and fan during mounting and/or assembly of the temperature modulation assembly. In some embodiments, the cover 800 can include one or more vents 808. In some examples, the vent 808 functions as an exhaust vent, allowing air that has circulated near the fins of the heatsink to flow out of the temperature modulation assembly. In some examples, the vent functions as an intake vent, allowing air to flow into the temperature modulation assembly before circulating near the fins of the heatsink. In some embodiments, the vents 808 can also be referred to herein as a plurality of vents 808 or, individually, e.g., each vent 808. In some embodiments, as shown, the vents 808 can include a pseudo-square shape as shown. In some embodiments, the vents 808 can include any suitable shapes, for example, rectangular, square, circular, among other shapes.

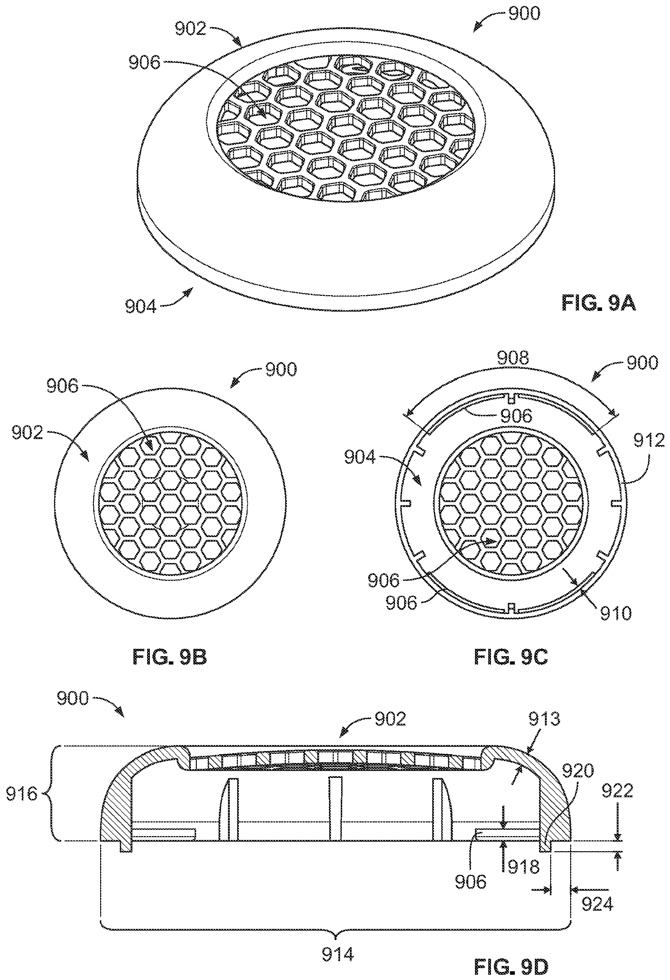

Referring again to FIG. 8A, in some embodiments, the cover 800 can include a locking mechanism 810. In some embodiments, the locking mechanism 810 can be shaped like a wedge. In some embodiments, the locking mechanism 810 can be configured to fit into and/or lock into with a corresponding feature of a cap. In some embodiments, the cover 800 can include alignment features 812. In some embodiments, the alignment features 812 can be configured to secure the cap in place, *e.g.,* the cap from FIGS. 9A-9D. In some embodiments, the alignment features 812 can include short walls that extend from the cover, e.g., without a wedge or perpendicular extended features in contrast to the locking mechanism 810. In some embodiments, the cover 800 can include a first and a second opening 814, 816. In some embodiments, the openings 814, 816 can be used for mounting the cover 800 to the spacer. In some embodiments, the first and second openings 814, 816 can be configured to receive a screw that can be used to mount into the top openings of the spacer of FIG. 5A and 5B and/or to the openings in the fan of FIG. 7.

Referring to FIG. 8B, a side view of the cover is presented, according to some embodiments. As shown, the cover 800 can include a locking mechanism 810 and an alignment feature 812. In some embodiments, cover 800 can include one or more vents 808 such as: a first vent 818, a second vent 820, a third vent 822 and a fourth vent 824. In some embodiments, the width of the first vent 818 can be greater than the width of the second, third and fourth vents 820, 822, 824. In some embodiments, the width of the second vent 820 can be less than the width of the first vent 818 but greater than the widths of the third and fourth vents 822, 824. In some embodiments, the third vent 824 can have a width less than the widths of the first and second vents 818, 820 and have a width greater than the width of the fourth vent 824. In some embodiments, the fourth vent 824 can have a width less than the widths of the first, second and third vents 818, 820, 822. In an example, the width of the first or most central vent 818 can be greater than the widths of the other vents 820, 822, 824, where each succeeding vent farther away from the central vent 818 can have a width that is less than the widths of the vents closer to the center. In an example, the width of the vents 818-824 can be in a range of approximately 3.00 - 5.00 mm.

Referring to FIG. 8C, a top view of the cover is presented, according to some embodiments. In some embodiments, the cover 800 can include a central opening 806 as discussed above. In some embodiments the first and second openings, 814, 816 from FIG. 8A are shown. As described above, the first and second openings 814, 816 can be configured to align with the top openings of the spacer of FIGS. 5A-5D. In some embodiments, the locking mechanism 810 and alignment feature 812 of FIG. 8A and 8B are also shown. The central opening 806 can have an additional notch 826 configured to let wires from the fan pass through underneath the cover. In some embodiments, the notch 826 of the central opening can extend by approximately 2.80 mm - 3.20 mm from the central opening 806. In some embodiments the central opening 806 can have a width 830 in a range of approximately 40 - 41 mm. In some embodiments the central opening 806 can have a width 830 of approximately 40.50 mm.

Referring to FIG. 8D, another side view of the cover is presented, according to some embodiments. As shown, the cover can be slightly tapered. In some embodiments, the cover can have a top width 832 that can be in range of approximately 62-64 mm and a bottom width 834 that can be in a range of approximately 63-65 mm. In some embodiments, the top width 832 can be approximately 63 mm and the bottom width 834 can be approximately 64 mm. In an example, the bottom width can be approximately l mm less than the top width. In some embodiments, the cover 800 can include one or more groups of vents 835, 837. In some embodiments, one or more groups of vents 835, 837 can be located along a wall portion 839 of the cover 800. In some embodiments, the one or more groups of vents 835, 837 can be located at separate, e.g., opposite, sides from another. In an example a first group of openings 835 can be located along one side of the wall portion 839 of the cover 800, separate and opposite from, a second group of vents 837, as shown. An exemplary locking mechanism is encircled in 834 and is further described in FIG. 8E.

Referring to FIG. 8E, a zoom-in view of a locking mechanism of the cover is presented, according to some embodiments. In some embodiments, the locking mechanism 810 can have a height 836 in a range of approximately 2.70 - 3.10 mm. In an example, the locking mechanism can have a height 836 of approximately 2.90 mm. In some embodiments, a wedge portion of the locking mechanism 810 can extend 838 outwardly from the cover 800 by a range of approximately 0.90 mm - 1.10 mm. In an example, wedge portion of the locking mechanism 810 can extend 838 outwardly from the cover 800 by approximately 1.00 mm. In some embodiments, the wedge portion of the locking mechanism 810 can have a height 840 in a range of approximately 1.20 - 1.40 mm. In an example, the wedge portion of the locking mechanism 810 can have a height 840 of approximately 1.30 mm.

Referring still again to FIGS. 8A-8E, in some embodiments, the cover 800 can include a material such as a plastic and/or resin, among other materials. In a particular example, the cover 800 can include Dupont 801 resin, Dupont 2801, Acrylonitrile butadiene styrene (ABS) plastic, among other materials.

Referring to FIGS. 9A-9D, various views of a cap are presented, according to some embodiments. In some embodiments, the cap can enclose the temperature modulation assembly and be configured to allow air to flow into the temperature modulation assembly for device cooling. In some embodiments, the cap can couple directly to the cover of FIGS. 8A-8E. FIG. 9A illustrates a cap of the temperature modulation assembly. FIG. 9B illustrates a top view of the cap. FIG. 9C illustrates a bottom view of the cap. FIG. 9D a cross-sectional view of the cap.

Referring to FIGS. 9A and 9B, multiple views of a cap are presented, according to some embodiments. In some embodiments, the cap 900 can include a top portion 902 and a bottom portion 904. In some embodiments, the cap 900 can be secured to the cover of FIGS. 8A-8E. In an example, the cap 900 can be secured by a snap fit. In some embodiments, the cap can include one or more openings 906. In some embodiments, the openings 906 of the cap 900 can be referred to as a plurality of openings 906 and/or referred to, individually, e.g., each opening 906. In some embodiments, the openings 906 of the cap 900 can be in a hexagon shape. In some embodiments, the openings 906 can be configured to provide structural support to the cap 900. In some embodiments, although the openings 906 can have a hexagon shape as shown, other shapes can be used for the openings 906. In some embodiments, the openings 906 can include a shape selected from the group consisting of a circular shape, a square shape, a polygonal shape, among other shapes. In some embodiments, the openings 906 can allow air to flow into the temperature therapy device. In an embodiment, the cap 900 may not reach or touch the top of a fan, *e.g.,* the fan from FIG. 7.

Referring to FIG. 9C, a bottom view of a cap is presented, according to some embodiments. The bottom portion 904 of the cap 900 is shown. In some embodiments, in this view, a receiving portion 906 of the cap 900 can be seen. In some embodiments, the receiving portion 906 of the cap 900 can have a corresponding feature to receive the locking mechanism of the cover from FIGS. 8A-8E. In some embodiments, the receiving portion 906 can include a receptacle. In some embodiments, the receiving portion 906 can be a shape that is opposite to the shape of the locking mechanism from FIGS. 8A-8E, *e.g.,* to a wedge shape. In some embodiments, the receiving portion 906 can extend 910 inward from an outer wall 912 of the cap 900. In some embodiments, the extension 910 of the receiving portion 906 can be in a range of approximately 0.70 mm - 0.90 mm. In some embodiments, the extension 910 of the receiving portion 906 can be approximately 0.80 mm. In some embodiments, the receiving portion 906 can have a curved length 908 in a range of approximately 95 - 105 mm. In some embodiments, the cap 900 can have a curved length 908 of approximately 100 mm.

Referring to FIG. 9D, a cross-sectional view of the cap from FIGS. 9A-9C is shown, according to some embodiments. In some embodiments, the cap 900 can have a width 914 of approximately 62 mm - 64 mm. In some embodiments, the cap 900 can have a width 914 of approximately 63 mm. In some embodiments, the cap 900 can have a height 916 of approximately 12 mm - 13 mm. In some embodiments, the cap 900 can have a height 916 of approximately 12.5 mm. In some embodiments, the receiving portion 906 can have a height 918 in a range of approximately 1.3 mm - 1.5 mm. In some embodiments, the receiving portion 906 can have a height 918 of approximately 1.5 mm. In some embodiments, the outer wall 912 can have a thickness 913 in a range of approximately 1.60 mm - 1.80 mm. In some embodiments, the outer wall 912 can have a thickness 913 of approximately 1.70 mm. In some embodiments, the bottom portion 904 of the cap 900 can include an extruded portion 920. In some embodiments, the extruded portion 920 can extend 922 outward away from the cap 900 by a range of approximately 1.40 mm - 1.60 mm. In some embodiments, the extruded portion 920 can extend 922 outward away from the cap 900 by approximately 1.50 mm. In some embodiments, the extruded portion 920 can be offset 924 from the outer wall 912 by a range of approximately 2.00 mm - 3.00 mm.

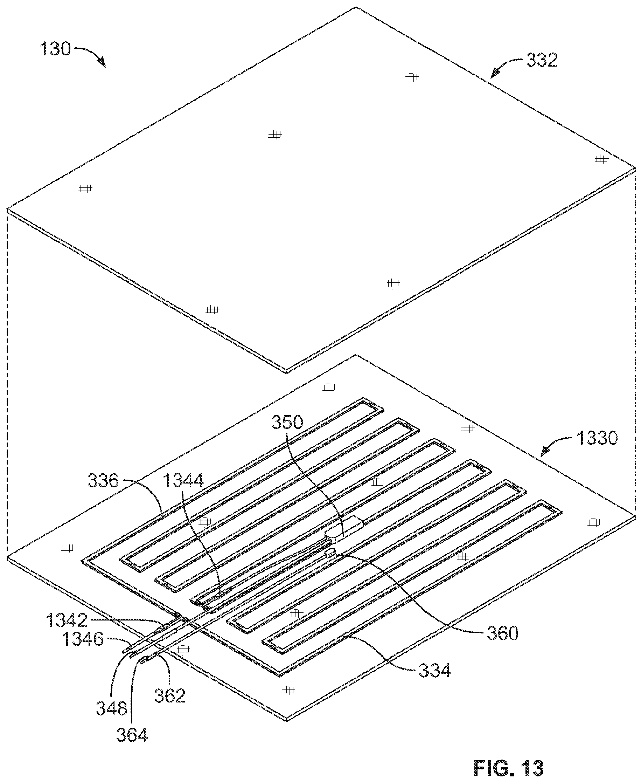

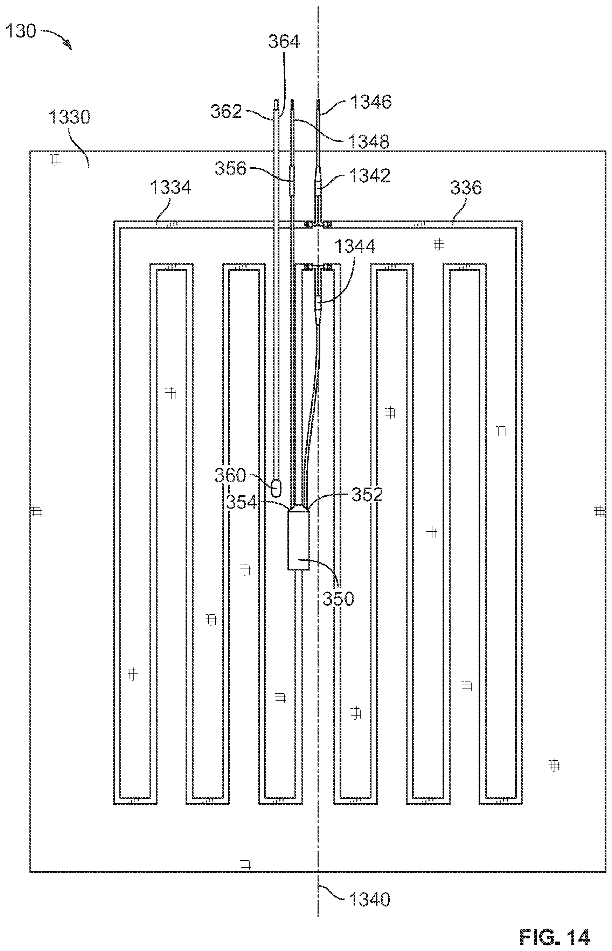

Referring to FIGS. 9A-9D, in some embodiments, the cap 900 can include a material such as a plastic and/or resin, among other materials. In a particular example, the cap 900 can include Dupont 801 resin, Dupont 2801, Acrylonitrile butadiene styrene (ABS) plastic, among other materials.In some embodiments, the temperature therapy module can include a heating generation unit. FIG. 13 illustrates an exploded upper perspective view of a heating generation unit, according to some embodiments. FIG. 14 illustrates a plan view of the electrical heating wire on the lower sheet of the heating generation unit, according to some embodiments.

As illustrated in FIGS. 13 and 14, in the illustrated embodiment, a heat generation unit 130 comprises a first (lower) rectangular sheet of cloth 1330 and a second (upper) rectangular sheet of cloth 332. Each sheet has outer dimensions of approximately 250 millimeters by approximately 200 millimeters. In the illustrated embodiment, each sheet comprises a 200g needle punch material (i.e., non-woven material formed by a conventional needle punching process) having a thickness of approximately 1.5 millimeters. The material has a density of approximately 200 grams per square meter. At least one electrical resistance wire is positioned between the two sheets. In the illustrated embodiment, a first resistance wire 334 and a second resistance wire 336 are secured to the upper surface of the lower sheet by lock stitching (not shown) in a conventional manner. The resistance wires can also be secured to the upper sheet in a similar manner. In one embodiment, each resistance wire comprises a thin, flat resistance wire, such as, for example, a commercially available titanium resistance wire. In the illustrated embodiment, the cross-sectional dimensions of the resistance wires are selected to provide a resistance of approximately 16 ohms per meter. Each resistance wire has a length of approximately 1.25 meters such that each wire has a total resistance of approximately 20 ohms.

The two resistance wires 334, 336 form two maze-like patterns, which are substantially symmetric about a centerline 1340 of the lower sheet 1330. Each resistance wire extends from a first common terminal 1342 to a second common terminal 1344 such that the two segments are connected in parallel. The first common terminal of the resistance wires is connected directly to a first supply wire 1346. The second common terminal of the resistance wires is connected to a second supply wire 1348 via a thermal cutoff switch 350. The thermal cutoff switch has a first terminal 352 connected to the second common terminal of the resistance wires and has a second terminal 354 connected to the second supply wire via a connector 356. The thermal cutoff switch 350 is normally closed such that the control unit 140 is electrically connected to the second common terminal 1344 of the resistance wires 1334, 1336. The first common terminal 1342 of the resistance wires is always connected to the control unit. Thus, current is conducted from the first terminal around each of the first resistance wire and the second resistance wire in parallel. Since each resistance wire has a resistance of approximately 20 ohms, each resistance wire generates approximately 14 watts of heat at a voltage of approximately 16.8 volts. The two resistance wires generate a total of approximately 28 watts of heat.

The thermal cutoff switch 350 is set to open the circuit when the temperature proximate to the thermal cutoff switch exceeds approximately 80 degrees Celsius +/-5 degrees and to stay open until the temperature reduces to approximately 55 degrees Celsius +/-10 degrees. In one embodiment, the thermal cutoff switch comprises a KLS-KSD9700 thermal fuse commercially available from Ningbo KLS Imp & Exp Co. Ltd. In Beilun Ningbo Zhejiang China. The thermal cutoff switch is positioned across portions of the heating wire such that the thermal cutoff switch directly senses the temperature of the heating wire and disconnects the electrical path well before the heat from the heating wire is communicated though the lower sheet and the material of the lower structure 112 to a user (not shown).

As further shown in FIGS. 13 and 14, a thermistor 360 is secured to the first (lower) sheet of cloth 1330. The thermistor is also positioned near the center of the first sheet; however, the thermistor is positioned between two adjacent segments of the first resistance wire 334 rather than directly on the resistance wire. A first wire 362 and a second wire 364 extend from the thermistor and are connected to the control unit 140. In one embodiment, the thermistor is a negative temperature coefficient (NTC) thermistor. For example, the thermistor may be an MF52-104F- 3950-600L thermistor commercially available from Dongguan Xinxiang Electronic Technology Co., Ltd., in China. The thermistor has a resistance that varies over a wide temperature range. For example, at 55 degrees Celsius, the thermistor has a resistance of approximately 29,733 ohms; at 60 degrees Celsius, the thermistor has a resistance of approximately 24,753 ohms; and at 71 degrees Celsius, the thermistor has a resistance of approximately 16,794 ohms. The resistance of the thermistor is readily detectable in a conventional manner to determine when the temperature of the thermistor exceeds a selected temperature.

After the thermal cutoff switch 350 and the thermistor 360 are positioned on the first (lower) sheet 1330, and after the first common terminal 1342 is connected to the first supply wire 1346 and the second common terminal 1344 is connected to a second supply wire 348, the second (upper) sheet 332 is secured to the first sheet. In the illustrated embodiment, the lower surface of the second sheet includes an adhesive to removably attach the second sheet to the first sheet.

Some non-limiting examples of a therapy device have been described. Additional embodiments of temperature therapy devices are described in U.S. Provisional Patent Application No. 63/090 987. Furthermore, some non-limiting examples of components of a therapy device have been described. Additional embodiments of such components, including flexible thermal spreaders (e.g., heat spreader 146, 300), heating and/or cooling elements (e.g., thermoelectric coolers (TECs) 150, 400), flexible substrates (e.g., flexible layers of a multi-layer retention mechanism 102), and coupling materials (e.g., adhesives, tapes, etc.) are also described in U.S. Provisional Patent Application No. 63/090,987.

Some embodiments of a therapy device including a thermoelectric cooler (TEC) have been described. A TEC is one example of a temperature control (e.g., heating and/or cooling) component that may be used in the temperature therapy device. In some embodiments, heating and/or cooling components other than a TEC may be used. For example, a Peltier device, a Peltier heater, a Peltier heat pump, or any other suitable heating and/or cooling component may be used.

### (ii) Compressive Therapy Module

In many instances, this disclosure describes the compressive therapy module (sometimes referred to herein as a compression pad) as being an inflatable bladder and/or a vibration pod; however, in general any element capable of applying a compressive force can be used (e.g., gel filled pockets, shape memory material, etc.).

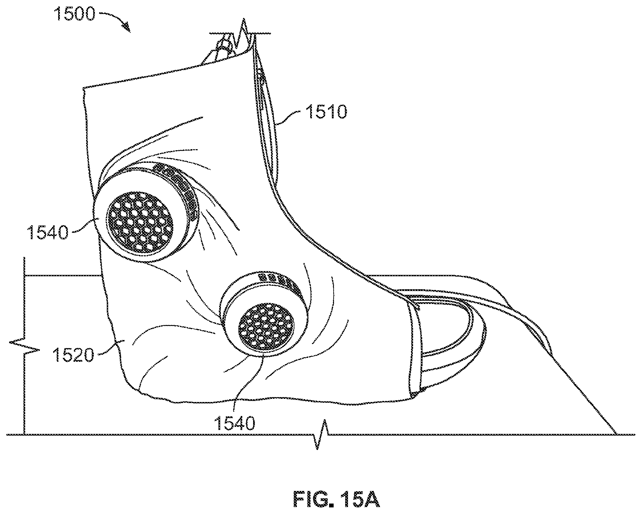

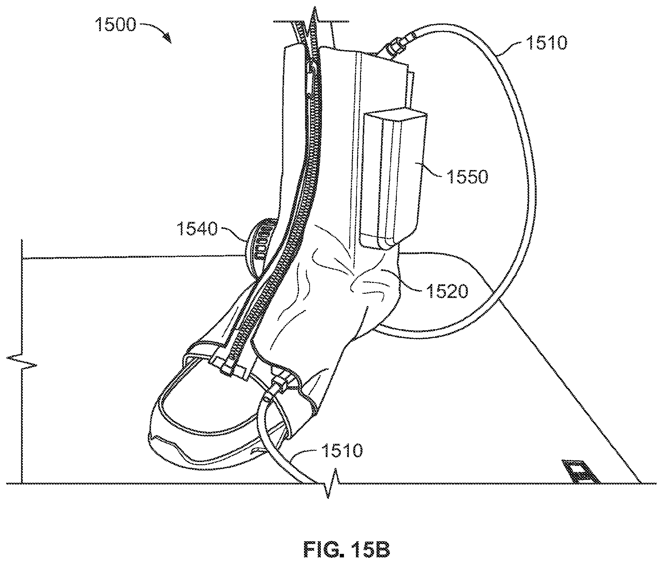

In general, the bladder is configured to effectively control the position of the therapy device relative to the user's body part, and uniformly apply compressive and/or thermal therapy to the user's body part. In some embodiments, the bladder is configured to substantially uniformly wrap around a body part of the user. In particular embodiments, the bladder is configured to wrap around a foot region the user (e.g., FIGS. 15A and 15B).

In some examples, the bladder includes one or more (e.g., 1, 2, 3, 4, or more) layers of air tight medium that can include portions that are bonded together (sometimes called stays) in a particular pattern. In contrast, implementations without a bladder can sometimes only partially surround a user's body part and/or include substantial air gaps between the users body part and the temperature therapy device.

In some embodiments, the bladder is positioned between the top layer and the bottom layer of a multi-layer retention mechanism.

In some embodiments, the bladder is configured to allow temperature from a temperature therapy module to substantially uniformly contact the users body part. In an example, portions of the bladder can be inflated, and once inflated, the bladder can compress against the bottom layer. Upon compression, the pressure applied by the bladder to the bottom layer can allow for the bottom layer 124 to uniformly surround the user's body part.

In some embodiments, the bladder is be bonded to the top layer and/or the bottom layer via an adhesive, and/or one or more locking features (e.g., snaps).

In some embodiments, bonding the bladder to the outer perimeter of the top layer and/or bottom layer enables the bladder to wrap around the user's body part *(e.g.,* a user's ankle) when the bladder inflates, as opposed to lifting off the user's body part and only constricting around the user's body part.

In some embodiments, the bladder material includes thermal polyurethane (TPU) material).

In some embodiments, the bladder is securely and/or removably attached to the top layer and/or bottom layer using a zipper attachment. For example, the zipper attachment can allow for the bladder to be removed, e.g., after unzipping the zipper attachment between the bladder and top layer and/or bottom layer.

In some embodiments, the bladder is operatively connected to at least one tube (e.g., tube 1510 in FIGS 15A and 15B). The tube can be used to input or remove air from the bladder so as to inflate and/or deflate the bladder. In some embodiment, the tube is operatively coupled to an air compressor. In some embodiments, the air compressor is part of, or located within, a control module as described herein. In some embodiments, the air compressor is located external to the control module.

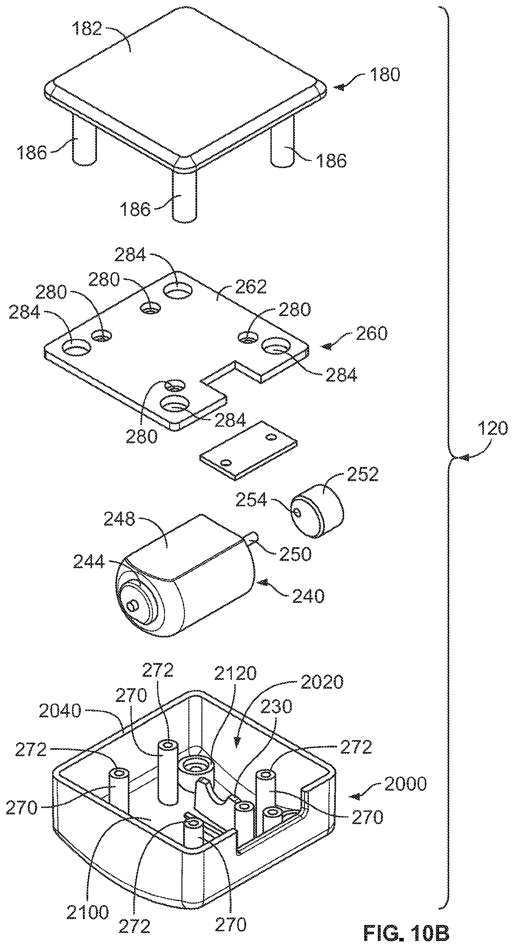

FIGS 10A and 10B illustrate a first vibration pod 120, according to some embodiments. In some embodiments, the therapy device includes a second, a third, a fourth, or more vibration pods that are identical or are substantially identical. The first vibration pod includes an upper cover 180. In the illustrated embodiment, a top surface 182 of the upper cover is square or substantially square with each side of the square having a length of approximately 45 millimeters). The upper cover has a thickness of approximately 4.25 millimeters to a lower surface 184. One or more protrusions 186 extend from the lower surface of the upper cover. Each protrusion has a diameter selected such that each protrusion fits through a selected one of the mounting bores 170 in the rear left set of mounting bores. For example, in the illustrated embodiment, the protrusions have a diameter of approximately 5 millimeters. Each protrusion has a length of approximately 16.5 millimeters. The end of each protrusion opposite the top of the upper cover has a central bore 188 that may be threaded to receive a machine screw (not shown). Alternatively, the central bore may be threadable to receive a self-taping screw.

The first vibration pod 120 can include a lower cover 2000 having a central cavity 2020. The lower cover can have a generally square upper surface 2040 surrounding the central cavity. In the illustrated embodiment, the peripheral dimensions of the upper surface of the lower cover generally correspond to the peripheral dimensions of the upper cover 180. The lower cover has an arcuate lower surface having one or more through bores 2060 formed therein. The through bores are spaced apart by distances corresponding to the spacing of the protrusions 186 of the upper cover 180. The through bores are counterbored with respect to the lower cover to receive the heads of the screws (not shown) that secure the lower cover to the upper cover.

A lower inner surface 2100 of the lower cover 2000 can correspond to the lower surface of the central cavity 2020. Each of the through bores 2060 can be surrounded by a respective inner protrusion 2120 that extends from the lower inner surface of the central cavity. The top surface of each inner protrusion can have a respective counterbore 2140 that surrounds the through bore and extends a selected distance into the protrusion. The diameter of each counterbore can be selected to correspond to the outer diameter of the protrusions 186 extending from the top cover 180 (e.g., approximately 5 millimeters in the illustrated embodiment) so that each protrusion of the top cover fits snugly into the respective counterbore of one of the inner protrusions of the lower cover. The depth of the counterbore in each inner protrusion in the central cavity is selected such that when the protrusions of the top cover are engaged with the counterbores, the lower surface 184 of the top cover is spaced apart from the upper surface 2040 of the bottom cover by a distance less than the thickness of the upper support structure 116. For example, in the illustrated embodiment, the two surfaces are spaced apart by approximately 1.85 millimeters, which is substantially less than the thickness (e.g., approximately 5 millimeters) of the upper support structure. Thus, when the top cover is secured to the bottom cover by the one or more screws (not shown) passing through the through bores 2060 of the lower cover and engaging the central bores 188 of protrusions extending from the upper cover, the portions of the upper support structure in contact with the upper cover and the lower cover are squeezed between the two covers to secure the first vibration pod 120 to the upper support structure. In some embodiments, a plurality of vibration pods are secured to the upper support structure in a like manner.

The lower inner surface 2100 of the lower cover 2000 includes a first motor bearing support 230 and a second motor bearing support 232. Each motor bearing support is sized and positioned to receive a respective motor bearing as described below. The lower inner surface further includes three raised ribs 234 positioned between the first and second bearing supports. Each rib has a respective upper surface positioned a selected distance from the lower inner surface.

The first bearing support 230 includes a generally semicircular upper surface sized to receive a front bearing 242 of a motor 240. The second bearing support 232 includes a generally semicircular upper surface sized to receive a rear bearing 244 of the motor. The motor has a generally horizontal lower surface 246 that rests on the three raised ribs 234 when the bearings of the motor are positioned in the respective bearing supports. The motor also has a generally horizontal upper surface 248, which is parallel to the upper surface in the illustrated embodiment. The motor includes a shaft 250. A front portion of the shaft extends from the front bearing to support an eccentric mass 252. The eccentric mass is positioned within an unobstructed portion of the inner cavity and is able to move freely within the portion of the cavity when the shaft of the motor is rotated.

The lower cover 2000 further includes a motor clamp plate 260 having an upper surface 262 and a lower surface 264. The motor clamp plate rests upon one or more clamp plate support protrusions 270 that extend upward from the lower inner surface 2100. Each clamp plate support protrusion has a respective central bore 272. Each central bore may be threaded to receive the threads of a machine screw (not shown). Alternatively, each central bore may be threadable by a self- tapping screw.

The motor clamp plate 260 is sized to fit within the lower cover 2000 and to rest upon the clamp plate support protrusions 270. The motor clamp plate includes one or more plate mounting through bores 280 that are aligned with the central bores of the clamp plate support protrusions. Each plate mounting through bore is counterbored on the upper surface 262 of the motor clamp plate so that the heads of the machine (or self-tapping) screws (not shown) do not extend above the upper surface of the motor clamp plate.

The lower surface 264 of the motor clamp plate 260 includes a respective protrusion 282 surrounding each plate mounting through bore 280. Each protrusion extends a short distance (e.g., approximately 2 millimeters; approximately 0.08 inch) below the lower surface. Each protrusion is counterbored to have an inside diameter corresponding to the outside diameter of a clamp plate support protrusion 270 (e.g., approximately 2.3 millimeters; approximately 0.09 inch in the illustrated embodiment). Thus, when the motor clamp plate is secured to the clamp plate protrusions, the motor clamp plate cannot shift laterally with respect to the lower cover.

The motor clamp plate 260 can further include one or more clearance through bores 284, which are positioned and sized to provide clearance for the one or more protrusions 186 that extend from the lower surface 184 of the upper cover 180. For example, in the illustrated embodiment, the clearance through bores have diameters of slightly greater than approximately 5 millimeters (approximately 0.2 inch) to provide a snug fit with respect to the protrusions.

The motor clamp plate 260 includes two motor engagement ribs 290 that extend from the lower surface 264. The engagement ribs are positioned to engage the generally horizontal upper surface 248 of the motor 240 when the motor clamp plate is positioned on the lower cover 2000 of the first vibration pod 120. The thickness of each rib with respect to the lower surface of the motor clamp plate is selected such that when the motor clamp plate is fully secured by the screws (not shown), the ribs are pressed against the horizontal upper surface of the motor. Accordingly, the motor is tightly secured between the ribs of the motor clamp plate and the three raised ribs 234 of the lower inner surface 2100 of the lower cover 2000.

In the illustrated embodiment, the motor 240 comprises a permanent magnet DC motor operating at approximately 5,300 revolutions per minute (RPM) from a 12-volt DC supply. In one embodiment, the motor comprises an FC130 style motor, which is commercially available from a number of sources. The motor draws approximately 0.09 Amperes at the rated RPM.

The motor 240 and the eccentric mass 252 together have an overall length of approximately 38 millimeters. The motor has an overall diameter of approximately 20.2 millimeters and is flattened to space the lower surface 246 and the upper surface 248 apart by approximately 15.4 millimeters.

The eccentric mass 252 is substantially cylindrical. The eccentric mass has an overall diameter of approximately 10 millimeters, and has a length along the shaft of the motor of approximately 7 millimeters. In the illustrated embodiment, the mass comprises powdered metal (e.g., iron), which is compacted to have a mass (weight) of approximately 3.5 grams. The eccentric mass is mounted on the shaft 250 of the motor 240 via a shaft bore 254 having a diameter of approximately 2.1 millimeters. In the illustrated embodiment, the shaft bore is offset from the center of the eccentric mass by approximately 2.2 millimeters to cause the mass to impart a vibration. The vibration is communicated from the shaft of the motor and through the bearings 242, 244 to bearing supports 230, 232 to cause the lower cover 2000 of the vibration pod 120 to vibrate.

### (iii) Control Unit

In general, one or more control units (or modules) can be operatively (e.g., electrically, pneumatically, and/or mechanically) connected with one or more modules (i) to (iv) (or a component thereof) using a wired or wireless connection.

In general, the control unit can include or house various electro-mechanical components to drive the system as described herein, including, for example, a miniature DC compressor, a plurality of (e.g., 2,3, 4, 5, 6, 7, 8, or more) solenoid valves (or manifolds), a pressure sensor, a battery, a battery charging interface (or power j ack), a printed circuit board (PCB), Bluetooth Low Energy (BLE) components, a user interface, a programming or debugging interface (e.g., Bluetooth, USB or other serial communication port for manufacturing and/or service). In particular embodiments, the battery charging interface is a USB-C charging port with a cover (e.g., rubber-like material) for ingress protection. The cover may be tethered to the enclosure of the USB-C charging port so that it doesn't get lost when the cover is removed (e.g., for accessing the USB-C port). In particular embodiments, the debugging interface is managed by a Bluetooth connection. In particular embodiments, the debugging interface cam be managed by a USB connection.

The control unit may be compact, light-weight, wireless, and/or Bluetooth-enabled.

In some embodiments, the control unit may comprise a length in a range from 0.5 inch to 6 inches (1.27 cm to 15.24 cm) and/or a weight in a range up to 8oz, 9oz, 10oz, 11oz, or 12 oz. (227 gr, 255 gr, 283 gr, 311 gr or 340 gr). In particular embodiments, the control unit comprises a size of 4.75 inches × 2.75 inches × 1 inch (12 cm x 7 cm x 2.54 cm).

In some embodiments, the control unit may be used to enable Bluetooth connection between a module and a mobile device using a mobile application. The Bluetooth feature may enhance user's experience. An example mobile application is described in U.S. Patent Application No. 17/372,237.

In some embodiments, a module (or a component thereof) synchronizes with another module (or a component thereof) by sending and/or receiving data in radio frequency signals and/or Bluetooth signals using the control unit(s).

In some embodiments, the control unit may include a box (e.g., box 1550 in FIG. 15B) to house a plurality of electro-mechanical components. The control unit may further include a display, one or more minimal user interfaces, including a pressure level button, a session time button, a Start / Stop button, a power button (or ON / OFF button) for a user to set personalized parameters (e.g., pressure level, session time, Start / Stop, or other suitable parameters) to control the operation of a module.

In some embodiments, biometric sensors and/or thermal sensors may be used additionally in the system, resulting in an electrical connection between the control unit and the air compression wrap.

In some embodiments, a mobile device (e.g., a smartphone) can replace and/or supplement the dedicated control unit.

### (iv) Combination of Multiple Modules

In some embodiments, the system and devices as described herein includes 2, 3, 4, or more above-mentioned modules (i) - (iv) for delivering, applying, and/or operating thermal and/or compressive therapy, as described further below.

In some embodiments, the therapy device includes an inner layer that contacts a body surface of the user.

In some embodiments, the therapy device further includes an outer layer.

In some embodiments, one or more therapy modules (e.g., a temperature modulation assembly, and/or a vibration pod) as described herein are operatively connected to and located on the outer layer of the therapy devices. In some embodiments, one or more therapy modules (e.g., a compression pad, a heat generation unit, a temperature modulation assembly, and/or a vibration pod) are securely located (e.g., enclosed) between the outer layer and the inner layer of the therapy device. Accordingly, thermal therapy and/or compression therapy can be delivered to the body surface of the user from the outer portion (e.g., outer layer or between the outer layer and the inner layer) of the therapy device without needing the therapy modules contact the body surface of the user and bring discomfort to the user.

In particular embodiments, the therapy device is a footwear.

FIGS. 15A and 15B illustrate a footwear 1500 that includes one or more (e.g., two) temperature modulation assemblies 1540 operatively attached to outer layer that encloses one or more compression pads 1520, so as to deliver at least one of thermal (hot and/or cold) and compressive therapies.

In some embodiments, the footwear further includes one or more tubes 1510 that are operatively connected to one or more compression pads 1520 so as to inflate and/or deflate the one or more compression pads during the treatment.

In some embodiments, the footwear is configured to include one or more openings. In some embodiments, an opening is located at the top portion of the footwear for receiving a foot from a user. In some embodiments, an opening is located at the bottom front portion of the footwear for providing mobility and/or comfort to the foot. In some embodiment, a tube 1510 is disposed in proximity to an opening of the footwear. For example, as shown in FIGS. 15A and 15B, at least one tube 1510 is disposed in proximity to one opening of the footwear 1500 and is operatively connected with the compression pad 1520.

In some embodiments, the footwear further includes a box 1550. The box 1550 can include a control unit, a battery pack, an user interface, and/or other components for operating the therapy device and/or communicating with the user.

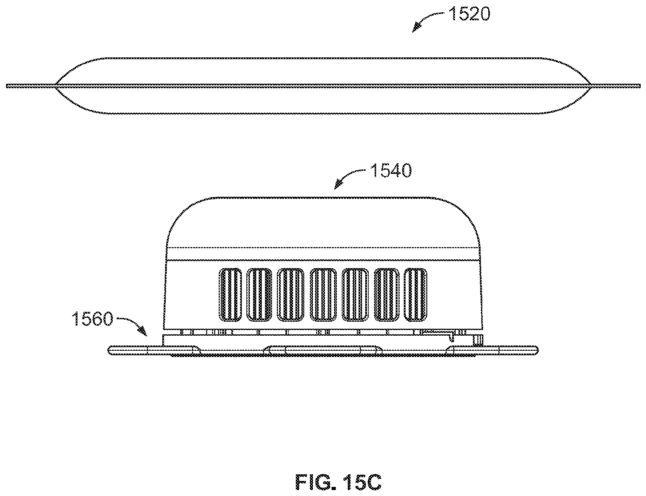

FIG. 15C illustrates components of a footwear, according to some embodiments. As shown in FIG. 15C, the footwear can include a compression pad (e.g., compression pad 1520) and a temperature modulation assembly 1540.

Referring to FIGS. 15A-15C, in some embodiments, the temperature modulation assembly 1540 is the same as the temperature modulation assembly 140 as described above. In some embodiments, the temperature modulation assembly 1540 includes a spacer 1560 that is the same as the spacer 550 as shown in FIGS. 5E-5G. In some embodiments, the spacer 1560 includes one or more (e.g., 1, 2, 3, 4, 5, 6, 7, 8, 9, 10, or more) wings that enables the bladder 1520 to push the temperature modulation assembly 1540 towards the user's body during the inflation of the bladder 1520. Thus, the temperature generated by the temperature modulation assembly 1540 can be delivered to the user's body. In some embodiments, 5 or more wings are preferable for stabilizing the temperature modulation assembly 1540 towards the user's body during the operation of the footwear (e.g., during the inflation of the bladder 1520).

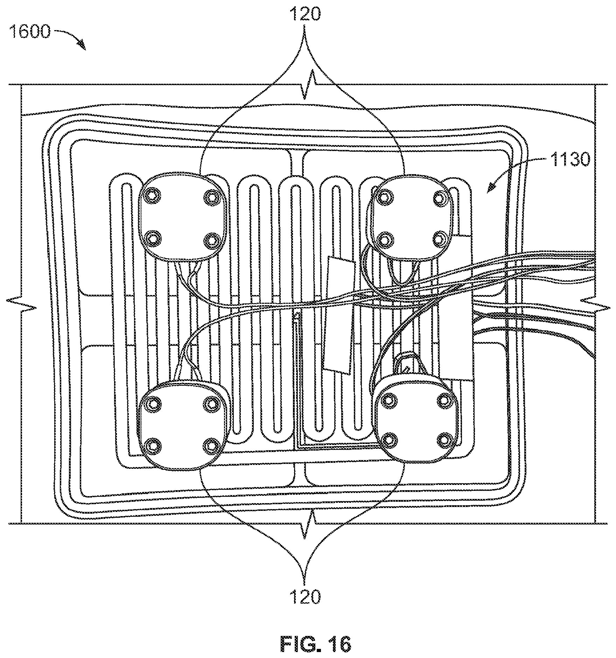

FIG. 16 illustrates a therapy device (e.g., a footwear) that includes one or more (e.g., four) compressive therapy modules (e.g., vibration pod 120) operatively attached to one or more temperature therapy modules (e.g., heat generation 130), so as to deliver at least one of thermal and compressive therapies.

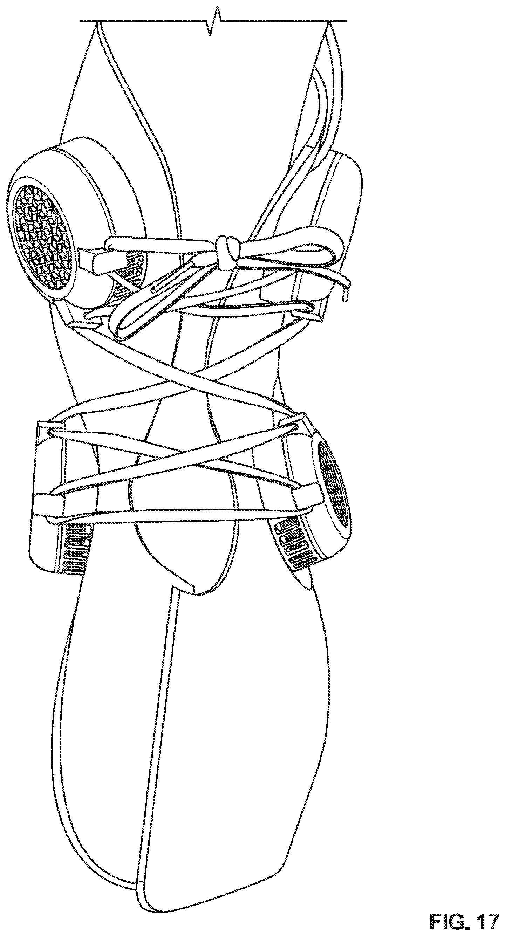

FIG. 17 illustrates a therapy device (e.g., a footwear) that includes one or more (e.g., four) temperature therapy modules (e.g., temperature modulation assembly 140) operatively attached to outer layer that may include or enclose one or more compressive therapy modules (e.g., a compression pad), so as to deliver at least one of thermal (hot and/or cold) and compressive therapies to the entire area (e.g., from front to back, from an upper to lower portion, and/or from outer to inner) of the foot and/or ankle of the user.

In some embodiments, the footwear includes a piece cloth to wrap around the foot area of the user, and further includes one or more hook and loop (e.g., products of Velcro^{®}) and shoe laces such that the footwear securely contacts the foot area of the user. Advantageously, the hook and loop and/or shoe laces can be stretchable for providing comfort to a user's foot. In some embodiments, the hook and loop and/or shoe laces can be replaced by one or more zippers.

### Computer Systems

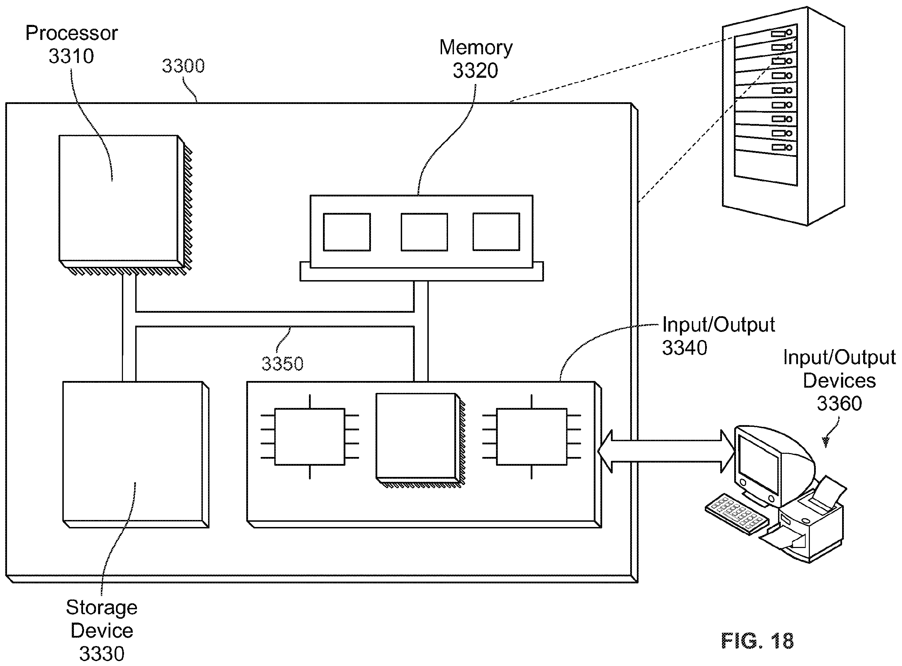

FIG. 18 is a block diagram of an example computer system 3300 that may be used in implementing the technology described in this document. General-purpose computers, network appliances, mobile devices, or other electronic systems may also include at least portions of the system 3300. The system 3300 includes a processor 3310, a memory 3320, a storage device 3330, and an input/output device 3340. Each of the components 3310, 3320, 3330, and 3340 may be interconnected, for example, using a system bus 3350. The processor 3310 is capable of processing instructions for execution within the system 3300. In some implementations, the processor 3310 is a single-threaded processor. In some implementations, the processor 3310 is a multi-threaded processor. The processor 3310 is capable of processing instructions stored in the memory 3320 or on the storage device 3330.

The memory 3320 stores information within the system 3300. In some implementations, the memory 3320 is a non-transitory computer-readable medium. In some implementations, the memory 3320 is a volatile memory unit. In some implementations, the memory 3320 is a non-volatile memory unit.

The storage device 3330 is capable of providing mass storage for the system 3300. In some implementations, the storage device 3330 is a non-transitory computer-readable medium. In various different implementations, the storage device 3330 may include, for example, a hard disk device, an optical disk device, a solid-date drive, a flash drive, or some other large capacity storage device. For example, the storage device may store long-term data (e.g., database data, file system data, etc.). The input/output device 3340 provides input/output operations for the system 3300. In some implementations, the input/output device 3340 may include one or more of a network interface devices, e.g., an Ethernet card, a serial communication device, e.g., an RS-232 port, and/or a wireless interface device, e.g., an 802.11 card, a 3G wireless modem, or a 4G wireless modem. In some implementations, the input/output device may include driver devices configured to receive input data and send output data to other input/output devices, e.g., keyboard, printer and display devices 3360. In some examples, mobile computing devices, mobile communication devices, and other devices may be used.

In some implementations, at least a portion of the approaches described above may be realized by instructions that upon execution cause one or more processing devices to carry out the processes and functions described above. Such instructions may include, for example, interpreted instructions such as script instructions, or executable code, or other instructions stored in a non-transitory computer readable medium. The storage device 3330 may be implemented in a distributed way over a network, for example as a server farm or a set of widely distributed servers, or may be implemented in a single computing device.

Although an example processing system has been described in FIG. 18, embodiments of the subject matter, functional operations and processes described in this specification can be implemented in other types of digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible nonvolatile program carrier for execution by, or to control the operation of, data processing apparatus. Alternatively or in addition, the program instructions can be encoded on an artificially generated propagated signal, e.g., a machine-generated electrical, optical, or electromagnetic signal that is generated to encode information for transmission to suitable receiver apparatus for execution by a data processing apparatus. The computer storage medium can be a machine-readable storage device, a machine-readable storage substrate, a random or serial access memory device, or a combination of one or more of them.

The term "system" may encompass all kinds of apparatus, devices, and machines for processing data, including by way of example a programmable processor, a computer, or multiple processors or computers. A processing system may include special purpose logic circuitry, e.g., an FPGA (field programmable gate array) or an ASIC (application specific integrated circuit). A processing system may include, in addition to hardware, code that creates an execution environment for the computer program in question, e.g., code that constitutes processor firmware, a protocol stack, a database management system, an operating system, or a combination of one or more of them.

A computer program (which may also be referred to or described as a program, software, a software application, a module, a software module, a script, or code) can be written in any form of programming language, including compiled or interpreted languages, or declarative or procedural languages, and it can be deployed in any form, including as a standalone program or as a module, component, subroutine, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable computers executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Computers suitable for the execution of a computer program can include, by way of example, general or special purpose microprocessors or both, or any other kind of central processing unit. Generally, a central processing unit will receive instructions and data from a read-only memory or a random access memory or both. A computer generally includes a central processing unit for performing or executing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few.

Computer readable media suitable for storing computer program instructions and data include all forms of nonvolatile memory, media and memory devices, including by way of example semiconductor memory devices, e.g., EPROM, EEPROM, and flash memory devices; magnetic disks, e.g., internal hard disks or removable disks; magneto optical disks; and CD-ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's user device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), e.g., the Internet.

The computing system can include clients and servers. A client and server are generally remote from each other and typically interact through a communication network. The relationship of client and server arises by virtue of computer programs running on the respective computers and having a client-server relationship to each other.

While this specification contains many specific implementation details, these should not be construed as limitations on the scope of what may be claimed, but rather as descriptions of features that may be specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In certain implementations, multitasking and parallel processing may be advantageous. Other steps or stages may be provided, or steps or stages may be eliminated, from the described processes. Accordingly, other implementations are within the scope of the following claims.

### DEFINITIONS

The phraseology and terminology used herein is for the purpose of description and should not be regarded as limiting.

Measurements, sizes, amounts, and the like may be presented herein in a range format. The description in range format is provided merely for convenience and brevity and should not be construed as an inflexible limitation on the scope of the invention. Accordingly, the description of a range should be considered to have specifically disclosed all the possible subranges as well as individual numerical values within that range. For example, description of a range such as 1-20 meters should be considered to have specifically disclosed subranges such as 1 meter, 2 meters, 1-2 meters, less than 2 meters, 10-11 meters, 10-12 meters, 10-13 meters, 10-14 meters, 11-12 meters, 11-13 meters, etc.

Furthermore, connections between components or systems within the figures are not intended to be limited to direct connections. Rather, data or signals between these components may be modified, re-formatted, or otherwise changed by intermediary components. Also, additional or fewer connections may be used. The terms "coupled," "connected," or "communicatively coupled" shall be understood to include direct connections, indirect connections through one or more intermediary devices, wireless connections, and so forth.

The term "approximately", the phrase "approximately equal to", and other similar phrases, as used in the specification and the claims (*e.g.,* "X has a value of approximately Y" or "X is approximately equal to Y"), should be understood to mean that one value (X) is within a predetermined range of another value (Y). The predetermined range may be plus or minus 20%, 10%, 5%, 3%, 1%, 0.1%, or less than 0.1%, unless otherwise indicated.

The term "treatment" or "therapy" refers to any act, hobby, task, program that relieves tension, the treatment of disease or disorders by some remedial, rehabilitating, or curative process, a curative power or quality, or psychotherapy.

The term "temperature therapy" is exchangeable with "thermal therapy" or similar terms.

The term "compressive therapy" is exchangeable with "compression therapy" or similar terms.

The term "bladder" or "inflatable bladder" refer to a bladder that can be air filled, and can be applied to specific area(s) of a person's body. In some embodiments, a bladder is inflatable and/or deflatable.

The term "module," "therapy module," or similar terms refer to a device, or a portion of a device for delivering therapies such as compressive therapy and/or thermal therapy.

The indefinite articles "a" and "an," as used in the specification and in the claims, unless clearly indicated to the contrary, should be understood to mean "at least one." The phrase "and/or," as used in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The use of "including," "comprising," "having," "containing," "involving," and variations thereof, is meant to encompass the items listed thereafter and additional items.

Use of ordinal terms such as "first," "second," "third," etc., in the claims to modify a claim element does not by itself connote any priority, precedence, or order of one claim element over another or the temporal order in which acts of a method are performed. Ordinal terms are used merely as labels to distinguish one claim element having a certain name from another element having a same name (but for use of the ordinal term), to distinguish the claim elements.

Having thus described several aspects of at least one embodiment of this invention, it is to be appreciated that various alterations, modifications, and improvements will readily occur to those skilled in the art. Such alterations, modifications, and improvements are intended to be part of this disclosure, and are intended to be within the scope of the invention. Accordingly, the foregoing description and drawings are by way of example only.

## Claims

1. A wearable therapy delivery device, the device comprising:
an inner layer configured to contact a body surface of a user;
an outer layer;
one or more temperature therapy modules (130) enclosed between the inner layer and the outer layer proximate the inner layer;
one or more compressive therapy modules (120) located proximate the one or more temperature therapy modules,
wherein the one or more compressive therapy modules are configured to force the one or more temperature therapy modules toward the body surface of the user; **characterised in that**
a spacer (550) disposed between the compressive therapy module and the temperature therapy module, the spacer having a plurality of wings (552) extending from the spacer, wherein each wing of the plurality of wings is equidistant from each other wing.

2. The device of claim 1, wherein the temperature therapy module comprises at least one of a thermoelectric cooler (TEC) (150, 400) and a heating generation unit (130).

3. The device according to any one of claims 1 or 2, wherein the temperature therapy module is configured to operate in a range from 100°F to 140°F (37.8 °C - 60 °C).

4. The device according to any one of claims 1 to 3, wherein the compressive therapy module comprises an inflatable bladder (1520).

5. The device according to any one of claims 1 to 4, wherein the compressive therapy module is configured to operate in a range from 120 psi to 200 psi (827 kPa - 1378 kPa).

6. The device according to any one of claims 1 to 5, wherein the each wing of the plurality of wings are arranged to extend from a perimeter of the spacer.

7. The device according to any one of claims 1 to 6, wherein the spacer comprises 5 wings.

8. The device according to any one of claims 1 to 7, further comprising a control unit (140) configured to control operation of the temperature therapy module and the compressive therapy module.

9. The device according to any one of claims 1 to 8, wherein the device comprises a footwear item (1500).

## Patentansprüche

1. Am Körper tragbare Therapieabgabevorrichtung, wobei die Vorrichtung Folgendes umfasst:
eine innere Schicht, die dazu ausgelegt ist, eine Körperoberfläche eines Benutzers zu berühren;
eine äußere Schicht;
ein oder mehrere Temperaturtherapiemodule (130), die zwischen der inneren Schicht und der äußeren Schicht in der Nähe der inneren Schicht eingeschlossen sind;
ein oder mehrere Kompressionstherapiemodule (120), die sich in der Nähe des einen oder der mehreren Temperaturtherapiemodule befinden,
wobei das eine oder die mehreren Kompressionstherapiemodule dazu ausgelegt sind, das eine oder die mehreren Temperaturtherapiemodule zur Körperoberfläche des Benutzers zu drücken; **dadurch gekennzeichnet, dass**
ein Abstandshalter (550) zwischen dem Kompressionstherapiemodul und dem Temperaturtherapiemodul angeordnet ist, wobei der Abstandshalter eine Vielzahl von Flügeln (552) aufweist, die sich von dem Abstandshalter erstrecken, wobei jeder Flügel der Vielzahl von Flügeln äquidistant zu jedem anderen Flügel vorliegt.

2. Vorrichtung nach Anspruch 1, wobei das Temperaturtherapiemodul mindestens eines von einem thermoelektrischen Kühler (TEC) (150, 400) und einer Erwärmungserzeugungseinheit (130) umfasst.

3. Vorrichtung nach einem der Ansprüche 1 oder 2, wobei das Temperaturtherapiemodul dazu ausgelegt ist, in einem Bereich von 100°F bis 140°F (37,8 °C - 60 C °) zu arbeiten.

4. Vorrichtung nach einem der Ansprüche 1 bis 3, wobei das Kompressionstherapiemodul eine aufblasbare Blase (1520) umfasst.

5. Vorrichtung nach einem der Ansprüche 1 bis 4, wobei das Kompressionstherapiemodul dazu ausgelegt ist, in einem Bereich von 120 psi bis 200 psi (827 kPa - 1378 kPa) zu arbeiten.

6. Vorrichtung nach einem der Ansprüche 1 bis 5, wobei jeder Flügel der Vielzahl von Flügeln so angeordnet ist, dass er sich von einem Umfangsrand des Abstandshalters erstreckt.

7. Vorrichtung nach einem der Ansprüche 1 bis 6, wobei der Abstandshalter 5 Flügel umfasst.

8. Vorrichtung nach einem der Ansprüche 1 bis 7, ferner umfassend eine Steuereinheit (140), die dazu ausgelegt ist, den Betrieb des Temperaturtherapiemoduls und des Kompressionstherapiemoduls zu steuern.

9. Vorrichtung nach einem der Ansprüche 1 bis 8, wobei die Vorrichtung einen Schuhwerkartikel (1500) umfasst.

## Revendications

1. Dispositif d'administration de thérapie portable, le dispositif comprenant :
une couche interne configurée pour mettre en contact une surface corporelle d'un utilisateur ;
une couche externe ;
un ou plusieurs modules de thérapie thermique (130) enfermés entre la couche interne et la couche externe à proximité de la couche interne ;
un ou plusieurs modules de thérapie compressive (120) situés à proximité du ou des modules de thérapie thermique,
le ou les modules de thérapie compressive étant configurés pour forcer le ou les modules de thérapie thermique vers la surface corporelle de l'utilisateur ;
**caractérisé en ce que**
un espaceur (550) disposé entre le module de thérapie compressive et le module de thérapie thermique, l'espaceur ayant une pluralité d'ailes (552) s'étendant à partir de l'espaceur, chaque aile de la pluralité d'ailes étant équidistante l'une de l'autre.

2. Dispositif selon la revendication 1, le module de thérapie thermique comprenant au moins l'un d'un refroidisseur thermoélectrique (TEC) (150, 400) et d'une unité de génération de chauffage (130).

3. Dispositif selon l'une quelconque des revendications 1 ou 2, le module de thérapie thermique étant configuré pour fonctionner dans une plage de 100 °F à 140 °F (37,8 °C à 60 °C).

4. Dispositif selon l'une quelconque des revendications 1 à 3, le module de thérapie compressive comprenant une vessie gonflable (1520).

5. Dispositif selon l'une quelconque des revendications 1 à 4, le module de thérapie compressive étant configuré pour fonctionner dans une plage de 120 psi à 200 psi (827 kPa à 1378 kPa).

6. Dispositif selon l'une quelconque des revendications 1 à 5, chaque aile de la pluralité d'ailes étant agencée pour s'étendre à partir d'un périmètre de l'entretoise.

7. Dispositif selon l'une quelconque des revendications 1 à 6, l'espaceur comprenant 5 ailes.

8. Dispositif selon l'une quelconque des revendications 1 à 7, comprenant en outre une unité de commande (140) configurée pour commander le fonctionnement du module de thérapie thermique et du module de thérapie compressive.

9. Dispositif selon l'une quelconque des revendications 1 à 8, le dispositif comprenant un article chaussant (1500).
